# EUROPÄISCHE PATENTANMELDUNG

(11) **EP 3 203 399 A1**
(43) Veröffentlichungstag der Anmeldung: **09.08.2017**
(21) Anmeldenummer: 16197372.2
(22) Anmeldetag: 27.07.2010
(51) Int. Cl.: G06F 19/00, A61J 7/04

(54) **ANZEIGEEINRICHTUNG FÜR EINEN SPENDER FÜR ARZNEIMITTELPORTIONEN SOWIE DEREN VERWENDUNG**

(30) Priorität: 30.07.2009 DE 102009036004; 21.10.2009 DE 102009050442
(62) Teilanmeldung aus: 10740531.8
(71) Anmelder: Bayer Intellectual Property GmbH, 40789 Monheim am Rhein (DE)
(72) Erfinder: Ranze, Heike, 12203 Berlin (DE); Kresse, Mayk, 14195 Berlin (DE); Leifeld, Sabine, 14129 Berlin (DE); Elliesen, Jörg, 13467 Berlin (DE); Weber, Göran, 32549 Bad Oeynhausen (DE); Reinhold, Tom, 48161 Münster (DE); Bazargani, Parviz, 22607 Hamburg (DE)
(74) Vertreter: BIP Patents

(57) **Zusammenfassung**

Zur sicheren und einfachen Kontrolle der Einnahme von Arzneimittelportionen Ta wird eine Anzeigeeinrichtung für einen Spender für Arzneimittelportionen Ta geschaffen, wobei die Arzneimittelportionen zu regelmäßig wiederkehrenden Referenz-Einnahmezeitpunkten einzunehmen sind und wobei die Anzeigeeinrichtung eine Anzeige 1 sowie eine elektronische Ansteuerung für die Anzeige umfasst, dadurch gekennzeichnet, dass die Anzeige ein erstes Visualisierungsmittel 30, 40 zur Darstellung einer ersten Zeitspanne zwischen einem ersten Referenz-Einnahmezeitpunkt und dem aktuellen Zeitpunkt umfasst und sich der erste Referenz-Einnahmezeitpunkt dadurch auszeichnet, dass die Arzneimittelportion bis zu dem ersten Referenz-Einnahmezeitpunkt nicht genommen worden ist.

## Beschreibung

Die vorliegende Erfindung betrifft eine Anzeigeeinrichtung für einen Spender für Arzneimittelportionen, wobei die Arzneimittelportionen zu regelmäßig wiederkehrenden Referenz-Einnahmezeitpunkten einzunehmen sind und die Anzeigeeinrichtung eine Anzeige sowie eine elektronische Ansteuerung für die Anzeige umfasst. Die vorliegende Erfindung betrifft ferner die Verwendung dieser Anzeigeeinrichtung zur Kontrolle der Einnahme von Arzneimittelportionen, insbesondere von Hormonpräparat-Portionen, vor allem von Kontrazeptiva-Portionen, aus einem Spender.

Arzneimittel müssen regelmäßig und möglichst zu einem vorgegebenen Zeitpunkt oder innerhalb eines vorgegebenen Zeitraums genommen werden, um deren Wirksamkeit garantieren zu können. Daher hat es nicht an Versuchen gefehlt, Hilfsmittel bereitzustellen, die an die Einnahme der Arzneimittel erinnern. Als weit verbreitetes Hilfsmittel für Personen, die an einem Tage mehrfach Arzneimittel nehmen müssen, werden Schachteln verwendet, die mehrfach unterteilt sind, und in deren Abteile die Arzneimittel für den jeweiligen Einnahmezeitraum, etwa für eine Einnahme am Morgen, eine Einnahme zu Mittag und eine Einnahme am Abend manuell eingefüllt werden. Somit kann die die Arzneimittel nehmende Person jeweils kontrollieren, ob zu dem jeweils vorgesehenen Zeitpunkt bereits eine Arzneimittelportion genommen wurde oder nicht. Allerdings sind diese Hilfsmittel fehleranfällig, weil die Person natürlich beispielsweise auch zu den vorgeschriebenen Zeitpunkten bzw. in den dafür vorgesehenen Zeiträumen daran denken muss, die Arzneimittelportionen zu nehmen oder überhaupt die Schachteln mit den Arzneimittelportionen zu füllen.

Für die Einnahme von Arzneimitteln sind daher Anzeigegeräte entwickelt worden, die an die Einnahme automatisch erinnern sollen. Beispielsweise ist in DE 100 35 5999 A1 eine Pillendose mit integriertem Timer beschrieben. Die Pillendose umfasst mehrere Fächer zur Aufnahme von Pillen, denen jeweils eine Leuchtdiode zugeordnet ist. Der Timer umfasst eine Digitalanzeige sowie Bedienknöpfe zu dessen Programmierung. Eine der Leuchtdioden wird zum jeweiligen vorprogrammierten Zeitpunkt der Einnahme der im zugeordneten Pillenfach enthaltenen Pillen angesteuert, so dass der Nutzer / die Nutzerin weiß, welche Pillen zu nehmen sind. Außerdem sind akustische und mechanische Signalgeber vorgesehen.

Weiterhin ist in DE 20 2004 012 232 U1 eine so genannte Pillenuhr beschrieben. Diese Pillenuhr weist zum einen eine Pillendose auf, die mit einer Digitaluhr kombiniert ist. Dadurch soll erreicht werden, dass der Nutzer / die Nutzerin die zu nehmenden Tabletten bei sich trägt, wenn er durch die Digitaluhr an die Einnahme erinnert wird. Die Pillendose weist sechs Fächer für die Tabletten auf. Die Digitaluhr ist mit einer Digitalanzeige für die aktuelle Uhrzeit sowie sechs weiteren Digitalanzeigen versehen, die in unterschiedlichen Modi an die Einnahme der Tabletten in den sechs Fächern der Pillendose erinnern. In einem Modus erinnert die Pillenuhr durch eine Countdown-Anzeige an die jeweilige Einnahme einer Tablette. Außerdem kann der Nutzer / die Nutzerin akustisch an die Einnahme erinnert werden. Die Pillenuhr kann wie eine Armbanduhr am Handgelenk getragen werden.

Weiterhin ist in DE 10 2004 023 641 A1 eine Vorrichtung mit Mitteln zum Verbinden mit einem Anti-Baby-Pillenblister offenbart. Die Vorrichtung dient zur Unterstützung der korrekten Einnahme von Anti-Baby-Pillen während des gesamten weiblichen Zyklus' und soll ferner gleichzeitig die Nichteinnahme-Phase berücksichtigen. Hierzu ist die Vorrichtung so ausgebildet, dass ein die Anti-Baby-Pillen enthaltender Blister mit der Vorrichtung verbunden werden kann, etwa über eine Klammer oder eine Tasche, und gegebenenfalls zusätzlich ein Detektionsmittel zum Einfügen des Blisters in die Vorrichtung und/oder zum Entfernen des Blisters von der Vorrichtung vorgesehen ist. Außerdem können sich Detektionsmittel zur Kontrolle des Füllzustandes der Tablettenpositionen im Blister befinden. Beispielsweise kann das Aufbrechen der Folie am Blister zur Entnahme einer Anti-Baby-Pille aus einer Tablettenposition einen Zeitgeber aktivieren. Die Vorrichtung weist ein Display in Form einer Digitaluhr auf, die den verbleibenden Zeitraum bis zum Beginn eines Alarmsignals anzeigt. Alarmsignale können über Lautsprecher, Vibratoren oder Leuchtdioden oder auch das Display abgegeben werden. Das Display zeigt der Nutzerin ferner an, an welchem Zyklustag sie sich gerade befindet.

In US 5,871,831 A eine ferner Vorrichtung gezeigt, die einen Computer mit einer Steckkontaktleiste zum Einschieben eines Tablettenblisters umfasst. Der Tablettenblister ist in geeigneter Weise so ausgebildet, dass sich an den Stellen auf der Folie, die bei der Entnahme einer Tablette durchstoßen werden, Leiterbahnen befinden, so dass diese beim Durchstoßen der Folie durchtrennt werden, wobei die Leiterbahnen beim Einstecken des Blisters in die Steckkontaktleiste am Computer mit den dort befindlichen Steckkontakten in elektrischen Kontakt gebracht werden. Dadurch kann die Entnahme einer Tablette automatisch registriert werden. Der Computer weist eine Digitalanzeige für die aktuelle Zeit und/oder den Zeitpunkt der nächsten Tabletteneinnahme auf. Außerdem können ein akustisches Signal oder LEDs an die Einnahme einer Tablette erinnern. Eine erste der LEDs dient zur Anzeige, dass eine Tablette genommen werden soll, eine zweite dazu, dass eine Einnahme noch zu früh wäre, und eine dritte dazu, dass der Einnahmezeitpunkt bereits überschritten ist.

Weiterhin ist in DE 102 17 929 A1 eine Vorrichtung zur Ausgabe von Tabletten offenbart. Diese Vorrichtung dient zur Aufnahme von Blistern und weist Drucktaster zum Ausdrücken einer Tablette aus dem Blister, ferner Mittel zur Einstellung von Einnahmezeiten sowie Mittel zur Anzeige der Einnahmezeiten auf. Die Vorrichtung erinnert über eine voreingestellte Einnahmezeit an die Einnahme einer Tablette. Hierzu dienen Alarmsymbole auf einer LCD-Anzeige. Mit der Vorrichtung wird darüber hinaus die Entnahme einer Tablette automatisch registriert.

Die vorstehend beschriebenen Vorrichtungen sind zwar geeignet, an den Einnahmezeitpunkt von Arzneimittelportionen automatisch zu erinnern. Hierzu dienen in den beschriebenen Spendern diverse unterschiedliche Anzeige- und Alarmmittel. In einzelnen Fällen wird an die gegebenenfalls zunächst vergessene oder bewusst übergangene Einnahme einer Arzneimittelportion wiederholt erinnert. Jedoch hat der Nutzer / die Nutzerin mit diesen Angaben keine vollständige Kenntnis über den Einnahmestatus. Beispielsweise ist es in der Regel nicht erforderlich, eine Arzneimittelportion zu einem präzise festgelegten Zeitpunkt zu nehmen. Vielmehr ist es ausreichend, die Arzneimittelportion innerhalb eines Zeitintervalls zu nehmen. Zwar ist in DE 20 2004 012 232 U1 hierzu angegeben, dass ein Alarm auch wiederholt werden kann, wobei der Nutzer / die Nutzerin einstellen kann, wie oft und in welchem zeitlichen Abstand der Alarm wiederholt werden soll. Jedoch wird nicht angegeben, in welcher Beziehung diese Alarmwiederholung zu der tatsächlichen Toleranz der Zeitüberschreitung sowie zur tatsächlichen Zeitüberschreitung steht.

Von daher besteht eine Aufgabe der vorliegenden Erfindung darin, eine Anzeigeeinrichtung für die Einnahme von sich in einem Spender befindenden Arzneimittelportionen zu schaffen, mit der die bekannten Nachteile behoben werden und mit der insbesondere gewährleistet sein soll, eine erhöhte Compliance bei der Einnahme der Arzneimittelportionen zu erreichen. Vor allem soll der Nutzer / die Nutzerin in die Lage versetzt werden, den aktuellen Status der Einnahme von Arzneimittelportionen schnell und einfach erkennen zu können. Insbesondere soll auch gewährleistet sein, dass die Einnahme insbesondere von Hormonpräparaten, vor allem von Kontrazeptiva, besonders sicher wird, indem dem Nutzer / der Nutzerin die erforderlichen Informationen über den aktuellen Einnahmestatus auf einen Blick visuell dargestellt werden. Weiterhin soll der Nutzer / die Nutzerin auch in die Lage versetzt werden, bei Fehlbedienungen sowie dann, wenn der Nutzer / die Nutzerin vergisst, die Arzneimittelportionen zu nehmen, schnell, einfach und ohne aufwändige Suche in Dokumentationen einen Vorschlag für Abhilfemöglichkeiten zu erhalten. Eine erhöhte Compliance bei der Arzneimittel-Einnahme soll auch dadurch erreicht werden, dass Fehlbedienungen weitgehend ausgeschlossen werden, etwa für den Fall, dass der Nutzer / die Nutzerin versehentlich mehr als eine Arzneimittelportion nimmt, diese Einnahme aber bei der Einnahmeverfolgung nicht berücksichtigt, oder eine Arzneimittelportion nicht nimmt, aber fälschlicherweise eine Einnahme bei der Einnahmeverfolgung verbucht. Ferner soll die Sicherheit der Einnahme auch langfristig gewährleistet sein, d.h. bei einer Einnahme von Arzneimitteln über einen langen Zeitraum.

Diese Aufgabe wird durch die Anzeigeeinrichtung gemäß Anspruch 1 sowie durch die Verwendung der Anzeigeeinrichtung nach Anspruch 23 gelöst. Bevorzugte Ausführungsformen der Erfindung sind in den Unteransprüchen angegeben.

Die erfindungsgemäße Anzeigeeinrichtung dient zur Kontrolle des Status' der Einnahme von Arzneimittelportionen durch einen Nutzer / eine Nutzerin. Vor allem soll der Status der Einnahme von Hormonpräparaten und ganz besonders von Steroid-Hormonpräparaten, beispielsweise zur Kontrazeption, angezeigt werden. Aber auch die Einnahme von Portionen von anderen Arzneimitteln soll einfach und sicher kontrollierbar sein, beispielsweise von Hormonpräparaten zur Hormonersatztherapie. Beispielsweise kann die Anzeigeeinrichtung zur Kontrolle des Status' von Arzneimittelportionen auch zur Behandlung aller chronischen Erkrankungen verwendet werden. Der Einsatz der Anzeigeeinrichtung ist dann ganz besonders wichtig, wenn die Arzneimittelportionen zu regelmäßig wiederkehrenden Referenz-Einnahmezeitpunkten einzunehmen sind. Daher soll die erfindungsgemäße Anzeigeeinrichtung vor allem zur Kontrolle des Status' der Einnahme von Kontrazeptiva dienen. In diesem Falle müssen die Arzneimittelportionen in einem regelmäßigen Einnahmeregime genommen werden, nämlich einmal pro Tag, wobei sich an eine mehrtägige Einnahmephase immer eine mehrtägige Unterbrechungsphase anschließt. Bei Nichtbefolgung der vorgeschriebenen Einnahmezeitpunkte kann die gewünschte Wirkung einer Kontrazeption ausbleiben.

Zum Erzielen des erfindungsgemäßen Zweckes weist die Anzeigeeinrichtung eine Anzeige sowie eine elektronische Ansteuerung für die Anzeige auf. In erfindungsgemäßer Art und Weise umfasst die Anzeige ein erstes Visualisierungsmittel zur Darstellung einer ersten Zeitspanne zwischen einem ersten Referenz-Einnahmezeitpunkt und dem aktuellen Zeitpunkt, wobei sich der erste Referenz-Einnahmezeitpunkt dadurch auszeichnet, dass bis zu dem ersten Referenz-Einnahmezeitpunkt keine Arzneimittelportion genommen worden ist (obwohl dies so vorgesehen war).

Als Referenz-Einnahmezeitpunkte werden im Sinne der vorliegenden Erfindung Zeitpunkte verstanden, an denen die Arzneimittelportionen idealer Weise genommen werden sollen, also beispielsweise bei täglich zu nehmenden Arzneimittelportionen an jedem Tag zum Beispiel um 7:00 oder auch um 9:00 oder zu einer anderen festen Uhrzeit, wobei es für eine rechtzeitige Arzneimittel-Einnahme jedoch in fast allen Fällen ausreicht, die Arzneimittelportion in einem Zeitintervall zu nehmen, das den Referenz-Einnahmezeitpunkt einschließt. Das Einnahmeintervall kann beispielsweise von einem Zeitpunkt 12 Stunden vor dem Referenz-Einnahmezeitpunkt bis zu einem Zeitpunkt beispielsweise 2 oder auch 24 Stunden nach dem Referenz-Einnahmezeitpunkt reichen. Bei beispielsweise täglich einmal zu nehmenden Arzneimittelportionen sind an jedem Tag ein derartiger Referenz-Einnahmezeitpunkt und das Einnahmeintervall zu bestimmen. Der Referenz-Einnahmezeitpunkt errechnet sich vorzugsweise aus dem Zeitpunkt einer ersten Entnahme einer Arzneimittelportion (aus einem Arzneimittelspender), insbesondere bevorzugt in einem Einnahmezyklus, und dem vorgeschriebenen Wiederhol-Rhythmus für die Einnahme. Die jeweiligen Referenz-Einnahmezeitpunkte errechnen sich dann aus den sich aus dem Wiederholrhythmus ergebenden jeweiligen Zeitabständen und dem Zeitpunkt der ersten Entnahme einer Arzneimittelportion aus dem Spender, der wiederum mit dem Zeitpunkt der ersten Einnahme einer Arzneimittelportion identisch ist. Somit liegen die Referenz-Einnahmezeitpunkte bei einmal am Tage zu nehmenden Arzneimittelportionen bei einer derartigen Berechnungsmethode bei jeweils n x 24 Stunden (n ist eine ganze Zahl) nach der ersten Entnahme der Arzneimittelportion aus dem Spender. Ist bei dieser Berechnungsmethode für die Referenz-Einnahmezeitpunkte im Falle einer täglich einmal zu nehmenden Arzneimittelportion eine erste Arzneimittelportion beispielsweise an einem ersten Tag um 12:00 aus dem Spender entnommen und damit von dem Nutzer / der Nutzerin genommen worden, so ist dieser Zeitpunkt (12:00) an jedem nachfolgenden Tag ein Referenz-Einnahmezeitpunkt, so dass an jedem diesem ersten Tag nachfolgenden weiteren Tag dann weitere Referenz-Einnahmezeitpunkte jeweils bei 12:00 liegen, zu denen die Arzneimittelportionen idealer Weise genommen werden sollen. Analog können die Referenz-Einnahmezeitpunkte bei einem jeweils zweimal am Tage zu nehmenden Arzneimittel beispielsweise dann, wenn eine erste Entnahme und damit Einnahme um 7:00 stattgefunden hat und eine zweite Einnahme 12 Stunden dazu versetzt stattfinden soll, an jedem nachfolgenden Tag zu 7:00 und 19:00 berechnet werden. Grundsätzlich können die Referenz-Einnahmezeitpunkte auch, ausgehend von der ersten Entnahme der Arzneimittelportion aus dem Spender, anders berechnet werden: Beispielsweise kann ein Referenz-Einnahmezeitpunkt aus den letzten zum Beispiel drei tatsächlichen Einnahmezeitpunkten berechnet werden.

Die Anzeigeeinrichtung weist somit eine Anzeige für eine Zeitüberschreitung des ersten Referenz-Einnahmezeitpunktes auf, d.h. eines Einnahmezeitpunktes, zu dem die Arzneimittelportion genommen werden sollte, der Nutzer bzw. die Nutzerin es jedoch versehentlich oder auch bewusst versäumt hat, die Arzneimittelportion bis zu diesem ersten Referenz-Einnahmezeitpunkt zu nehmen.

Indem die erfindungsgemäße Anzeigeeinrichtung die vorgenannten ersten Visualisierungsmittel zur Anzeige einer Zeitüberschreitung des ersten Referenz-Einnahmezeitpunktes aufweist, wird eine umfassende Kontrolle des Einnahmestatus' des Arzneimittels möglich: Die Anzeige einer Zeitüberschreitung ermöglicht es dem Nutzer / der Nutzerin oder auch einem Arzt, gegebenenfalls Abhilfemaßnahmen einzuleiten, um das Fehlen der Einnahme auszugleichen. Ferner ist es für den Nutzer / die Nutzerin auch sofort erkennbar, wann das Zeitintervall für die Einnahme abläuft, wenn er/sie das Zeitintervall kennt, in dem eine Arzneimittelportion genommen werden muss. Das Zeitintervall kann in dem Visualisierungsmittel natürlich auch separat dargestellt sein, so dass der Nutzer / die Nutzerin auf einen Blick erkennt, ob er/sie die Arzneimittelportion trotz Zeitüberschreitung des ersten Referenz-Einnahmezeitpunktes noch rechtzeitig, d.h. innerhalb des Zeitintervalls nehmen kann.

Die erfindungsgemäße Anzeigeeinrichtung ist vorzugsweise für die Einnahme von Kontrazeptiva und in diesem Falle ganz besonders in den Fällen vorteilhaft, in denen die Nutzerin einem so genannten flexiblen Einnahmeregime folgt. Kontrazeptiva müssen typischerweise täglich einmal genommen werden, wobei bei herkömmlichen Einnahmeregimes während einer Einnahmephase von 21 Tagen täglich eine Arzneimittelportion genommen wird und sich danach eine 7 Tage währende Unterbrechungsphase anschließt. Anschließend wiederholt sich dieser Einnahmezyklus wieder. Alternativ können die Einnahmephase auch 24 Tage und die Unterbrechungsphase 4 Tage andauern. Es ist auch bereits vorgeschlagen worden, Kontrazeptions-Präparate nach einem so genannten flexiblen Einnahmeregime zu nehmen. In diesem Falle dauert die Einnahmephase mindestens 24 Tage (obligatorische Einnahmephase) und höchstens beispielsweise 120 Tage an. Eine Entscheidung über die Dauer der Einnahmephase wird in den genannten Grenzen der Nutzerin überlassen, d.h. die Nutzerin kann nach Ablauf der obligatorischen Einnahmephase selbst entscheiden, ob sie die Unterbrechungsphase einleitet. Allerdings muss sie dies bis zum Ablauf der Maximalzeit der flexiblen Phase tun, d.h. im genannten Fall bis zum Ablauf von 120 Tagen. An die flexible Einnahmephase schließt sich typischerweise eine Unterbrechungsphase von 4 Tagen an.

Falls es die Nutzerin versäumt hat, eine Arzneimittelportion zu nehmen, kann sie die Überschreitung des Einnahmezeitintervalls zumindest dann als Übergang in eine Unterbrechungsphase nutzen, wenn die Einnahmephase bereits seit mindestens 24 Tagen angedauert hat (vor Ablauf der obligatorischen Einnahmephase würde die Einleitung einer Unterbrechungsphase dazu führen, dass die Kontrazeption nicht mehr gesichert ist). Insbesondere kann diese erste Zeitspanne der Zeitüberschreitung nach dem ersten Referenz-Einnahmezeitpunkt in diesem Falle als Zeitraum innerhalb der Unterbrechungsphase gerechnet werden. Die vorliegende Erfindung dient in diesem Falle daher außerdem dazu, der Nutzerin eine einfache Möglichkeit zur Entscheidung zu geben, von der Einnahmephase in die Unterbrechungsphase überzuwechseln: Wenn beispielsweise das Einnahmezeitintervall überschritten ist, kann die Nutzerin entscheiden, eine Unterbrechungsphase zu starten, damit die reguläre Einnahme nach der Einnahmeunterbrechung wieder ordnungsgemäß gestartet werden kann. Dies wäre mit den herkömmlichen Vorrichtungen zur Kontrolle der Einnahme nur dann möglich, wenn die Nutzerin theoretisch mit höchstmöglicher Selbstorganisation die Einhaltung des Einnahmeregimes kontrollieren würde. Denn die Nutzerin müsste in diesem Falle die Übersicht über die Zeitüberschreitung haben. Dies ist aber unter den üblichen und den alltäglichen Ablenkungen geschuldeten Bedingungen nicht ohne weiteres gegeben.

Somit ist mit der Anzeige der Zeitüberschreitung des ersten Referenz-Einnahmezeitpunktes eine erhöhte Sicherheit bei der Einnahme von Arzneimittelportionen erreichbar.

In einer bevorzugten Ausführungsform der Erfindung umfasst die Anzeige zusätzlich ein zweites Visualisierungsmittel zur Darstellung einer zweiten Zeitspanne zwischen einem zweiten Referenz-Einnahmezeitpunkt und dem aktuellen Zeitpunkt. In diesem Falle zeichnet sich der zweite Referenz-Einnahmezeitpunkt dadurch aus, dass er in einem vorgegebenen Einnahmezeitintervall liegt und eine Arzneimittelportion in dem vorgegebenen Einnahmezeitintervall genommen worden ist. Die zweite Zeitspanne erstreckt sich maximal bis zum dem zweiten Referenz-Einnahmezeitpunkt zeitlich nachfolgenden ersten Referenz-Einnahmezeitpunkt. D.h. bei vollständiger Skalenausnutzung des zweiten Visualisierungsmittels erstreckt sich die Anzeige über den gesamten Zeitraum zwischen dem zweiten Referenz-Einnahmezeitpunkt und dem ersten Referenz-Einnahmezeitpunkt für den Fall, dass die Arzneimittelportion bis zum ersten Referenz-Einnahmezeitpunkt nicht genommen worden ist oder genau zu diesem Zeitpunkt genommen wird. Wenn die Arzneimittelportion nicht bis zum Erreichen des ersten Referenz-Einnahmezeitpunktes genommen worden ist, wird das erste Visualisierungsmittel gestartet, das dann die sich anschließende erste Zeitspanne seit dem ersten Referenz-Einnahmezeitpunkt anzeigt. Die Darstellung der abgelaufenen zweiten Zeitspanne zwischen dem zweiten Referenz-Einnahmezeitpunkt und dem ersten Referenz-Einnahmezeitpunkt bleibt in diesem Falle vorzugsweise erhalten, kann aber in solchen Fällen auch gelöscht sein, in denen beispielsweise nicht der seit dem zweiten Referenz-Einnahmezeitpunkt bereits abgelaufene Zeitraum sondern der noch bis zum nächsten Referenz-Einnahmezeitpunkt verbliebene Zeitraum angezeigt wird.

Ebenso wie der erste Referenz-Einnahmezeitpunkt stellt auch der zweite Referenz-Einnahmezeitpunkt einen Zeitpunkt im Einnahmeregime dar, zu dem eine Arzneimittelportion idealer Weise genommen werden soll. Der Unterschied besteht nur insofern, als eine Arzneimittelportion im Falle des zweiten Referenz-Einnahmezeitpunktes genommen wurde und im Falle des ersten Referenz-Einnahmezeitpunktes nicht. Insofern gilt die oben angegebene Erläuterung für Referenz-Einnahmezeitpunkte auch hier.

Durch das zweite Visualisierungsmittel wird die zweite Zeitspanne dargestellt, die seit dem jüngsten zweiten Referenz-Einnahmezeitpunkt einer Arzneimittelportion bei ordnungsgemäßer Arzneimittel-Einnahme verstrichen ist. Somit kann der Nutzer / die Nutzerin eine zusätzliche Information über den aktuellen Status der Einnahme von Arzneimittelportionen bei ordnungsgemäßer Befolgung der Einnahmevorschriften erhalten. Indem der Nutzer / die Nutzerin somit bei vorschriftsmäßiger Einnahme verfolgen kann, welcher Zeitraum seit dem jüngsten zweiten Referenz-Einnahmezeitpunkt verstrichen ist, kann er/sie sich auf die nächste Arzneimittel-Einnahme einrichten.

Das zweite Visualisierungsmittel wird vorzugsweise durch eine erste Entnahme einer Arzneimittelportion aus dem Spender in einem Einnahmezyklus gestartet. Der zweite Referenz-Einnahmezeitpunkt und somit auch der erste Referenz-Einnahmezeitpunkt werden daher vorzugsweise für einen aktuellen Einnahmezyklus festgelegt und definiert. Ein Einnahmezyklus ist beispielsweise durch die Gesamtheit aller Einnahmevorgänge während einer Erkrankung festgelegt. Im Falle einer kontinuierlichen Einnahme etwa zur Hormonersatztherapie ist der Einnahmezyklus ebenfalls durch eine erste Entnahme gekennzeichnet und im Falle der Kontrazeption durch eine Einnahme- und eine Unterbrechungsphase, bevor eine neue Einnahmephase beginnt. Mit der ersten Entnahme einer Arzneimittelportion aus einem Spender in einem Einnahmezyklus werden somit die Referenz-Einnahmezeitpunkte festgelegt.

Indem die Referenz-Einnahmezeitpunkte jeweils vorzugsweise nur für einen einzigen Einnahmezyklus festgelegt werden, kann der Nutzer / die Nutzerin die Referenz-Einnahmezeitpunkte in einem neuen Zyklus erneut festlegen. Dies ist dann vorteilhaft, wenn sich der ursprünglich gewählte Referenz-Einnahmezeitpunkt für den Nutzer / die Nutzerin als unpraktisch herausgestellt hat und der Nutzer / die Nutzerin einen geänderten Referenz-Einnahmezeitpunkt wünscht.

Während die zweiten Referenz-Einnahmezeitpunkte durch die erste Entnahme einer Arzneimittelportion aus dem Spender festgelegt werden, ergeben sich die ersten Referenz-Einnahmezeitpunkte dadurch, dass bis zum dem zweiten Referenz-Einnahmezeitpunkt zeitlich nachfolgenden ersten Referenz-Einnahmezeitpunkt keine Arzneimittelportion genommen worden ist. In diesem Falle wird das erste Visualisierungsmittel somit durch Erreichen des ersten Referenz-Einnahmezeitpunktes gestartet, d.h. wenn bis zu diesem ersten Referenz-Einnahmezeitpunkt keine Arzneimittelportion genommen worden ist. In diesem Falle schließt also an die dargestellte zweite Zeitspanne seit dem zweiten Referenz-Einnahmezeitpunkt die erste Zeitspanne an, die seit dem ersten Referenz-Einnahmezeitpunkt verstrichen ist. In diesem Falle zeigen gleichzeitig die ersten und die zweiten Visualisierungsmittel den Zeitraum seit dem jüngsten zweiten Referenz-Einnahmezeitpunkt an, d.h. seit einem Referenz-Einnahmezeitpunkt, der dadurch gekennzeichnet ist, dass er in einem vorgegebenen Einnahmezeitintervall liegt, in dem die Arzneimittelportion genommen worden ist und daher eine ordnungsgemäße Einnahme stattgefunden hat.

Wenn eine Arzneimittelportion genommen wird, wird ein neuer zweiter Referenz-Einnahmezeitpunkt generiert. Wenn in diesem Falle lediglich das zweite Visualisierungsmittel die zweite Zeitspanne seit dem (bisherigen) zweiten Referenz-Einnahmezeitpunkt angezeigt hat (weil der nominale Zeitraum zur Einnahme der Arzneimittelportion noch nicht abgelaufen ist), wird die Anzeige der zweiten Zeitspanne gelöscht, und es wird eine neue zweite Zeitspanne angezeigt, die die Zeitdifferenz zwischen dem aktuellen Zeitpunkt und dem neuen zweiten Referenz-Einnahmezeitpunkt wiedergibt. Diese zweite Zeitspanne ist in letzterem Falle negativ, d.h. es wird zunächst die zweite Zeitspanne zwischen dem aktuellen Zeitpunkt und dem noch nicht erreichten neuen zweiten Referenz-Einnahmezeitpunkt angezeigt. Alternativ kann die zweite Zeitspanne in diesem Falle auch in Form des noch nicht abgelaufenen Zeitraums bis zum nächsten Referenz-Einnahmezeitpunkt angezeigt werden. Da die zweite Zeitspanne bis zum nächsten Referenz-Einnahmezeitpunkt länger als eine Einnahmeperiode ist, wird die noch nicht abgelaufene Zeitspanne vorzugsweise in zwei Skalenteilen angezeigt: einem ersten Skalenteil, der dem Restzeitraum bis zum bisherigen Referenz-Einnahmezeitpunkt reicht, und einem zweiten Skalenteil, der der regulären zweiten Zeitspanne entspricht. Somit wird im weiteren Verlauf dann vorzugsweise zunächst der über die reguläre Anzeige einer Einnahmeperiode hinausgehende Bereich sukzessive abgeschaltet und erst anschließend der reguläre Bereich.

War der nächste Referenz-Einnahmezeitpunkt zum Zeitpunkt der Entnahme einer Arzneimittelportion dagegen bereits erreicht, und wurde daher ein erster Referenz-Einnahmezeitpunkt generiert, so dass auch das erste Visualisierungsmittel eine erste Zeitspanne seit diesem (neu generierten) ersten Referenz-Einnahmezeitpunkt anzeigt, so wird die Anzeige der ersten Zeitspanne durch die Entnahme einer Arzneimittelportion gelöscht, und es wird eine neue zweite Zeitspanne angezeigt, die die Zeitdifferenz zwischen dem aktuellen Zeitpunkt und dem neuen zweiten Referenz-Einnahmezeitpunkt wiedergibt. Da die Arzneimittelportion in diesem Falle erst entnommen wurde, nachdem der nächste Referenz-Einnahmezeitpunkt bereits passiert war, ohne dass eine Arzneimittelportion genommen wurde, wird das zweite Visualisierungsmittel eine bereits verstrichene zweite Zeitspanne seit dem neuen zweiten Referenz-Einnahmezeitpunkt anzeigen. In einer möglichen Verfahrensweise wird der zweite Referenz-Einnahmezeitpunkt in Fällen, in denen der bisherige zweite Referenz-Einnahmezeitpunkt erheblich überschritten wurde, auf einen neuen zweiten Referenz-Einnahmezeitpunkt gesetzt, der um zwei Einnahmeperioden zwischen jeweils zwei aufeinander folgenden Referenz-Einnahmezeitpunkten verschoben ist und nicht nur um eine, d.h. in diesem Falle wird die Arzneimitteleinnahme einmal ausgesetzt. Alternativ und bevorzugt wird die Anzeige bei der Entnahme einer Arzneimittelportion aber nur um eine Einnahmeperiode versetzt, so dass der Nutzer / die Nutzerin dazu angehalten wird, noch eine weitere Arzneimittelportion zu entnehmen, um den ordnungsgemäßen Einnahmerhythmus wieder aufzunehmen.

In einer weiter bevorzugten Ausführungsform der Erfindung wird ein neuer zweiter Referenz-Einnahmezeitpunkt bei einer vorzeitigen Entnahme einer Arzneimittelportion nur dann erzeugt, wenn die Arzneimittelportion frühestens innerhalb des Einnahmezeitintervalls liegt. Falls der Entnahmezeitpunkt noch vor dem Beginn des Einnahmezeitintervall liegt, wird die Entnahme nicht als Arzneimitteleinnahme berücksichtigt, so dass es bei der bisherigen Anzeige der zweiten Zeitspanne verbleibt, denn in diesem Falle wird ein neuer zweiter Referenz-Einnahmezeitpunkt nicht generiert. Wird ein Arzneimittel dagegen innerhalb des Einnahmezeitintervalls vor dem Erreichen des zweiten Referenz-Einnahmezeitpunktes genommen, wird ein neuer zweiter Referenz-Einnahmezeitpunkt generiert. In diesem Falle wird zunächst eine neue negative zweite Zeitspanne angezeigt, d.h. die Zeitspanne zwischen dem aktuellen Zeitpunkt und dem später liegenden neuen zweiten Referenz-Einnahmezeitpunkt. Ein neuer zweiter Referenz-Einnahmezeitpunkt wird, wie oben erläutert, auch generiert, wenn das Arzneimittel erst nach dem Ablauf des Einnahmezeitintervalls genommen wird.

In einer weiter bevorzugten Ausführungsform der Erfindung sind das erste Visualisierungsmittel in mindestens einem ersten Anzeigebereich und das zweite Visualisierungsmittel in mindestens einem zweiten Anzeigebereich der Anzeige angeordnet.

Besonders bevorzugt ist es, wenn der mindestens eine erste Anzeigebereich und der mindestens eine zweite Anzeigebereich zueinander benachbart sind.

Weiter bevorzugt ist es, wenn das erste Visualisierungsmittel durch mindestens ein erstes Anzeigeelement gebildet ist.

Weiter bevorzugt ist es, wenn mindestens zwei erste Anzeigeelemente vorgesehen sind und die ersten Anzeigeelemente mit fortschreitendem Zeitablauf sukzessive angeschaltet werden. In diesem Falle stellen die ersten Anzeigeelemente den Zeitablauf graphisch und nicht in Form einer Ziffernanzeige dar.

Die ersten Anzeigeelemente können auch eine Ziffernanzeige darstellen. Beispielsweise kann die Ziffernanzeige die Anzahl der noch zu nehmenden Arzneimittelportionen anzeigen. Es können entweder die graphisch darstellenden ersten Anzeigeelemente oder die Ziffern anzeigenden ersten Anzeigeelemente oder beide vorhanden sein.

Weiter bevorzugt ist es, wenn das zweite Visualisierungsmittel durch mindestens ein zweites Anzeigeelement gebildet ist.

Weiter bevorzugt ist es, wenn mindestens zwei zweite Anzeigeelemente vorgesehen sind und die zweiten Anzeigeelemente mit fortschreitendem Zeitablauf sukzessive angeschaltet oder sukzessive abgeschaltet werden. Somit stellen die zweiten Anzeigeelemente den Zeitablauf vorzugsweise graphisch und nicht in Form einer Ziffernanzeige dar. Für den Fall des sukzessiven Abschaltens der Anzeigeelemente mit fortschreitendem Zeitablauf werden zunächst alle Anzeigeelemente oder zumindest die Anzeigeelemente für Zeitabschnitte, die bis zum nächsten Referenz-Einnahmezeitpunkt verbleiben, angezeigt und diese dann mit fortschreitendem Zeitablauf abgeschaltet.

Durch die graphischen Darstellungen ist eine schnelle, einfache und fehlerfreie Erkennbarkeit der ersten bzw. der zweiten Zeitspanne gegeben.

Weiter bevorzugt ist es, wenn jedes des mindestens einen ersten und/oder jedes des mindestens einen zweiten Anzeigeelements im Falle der graphischen Darstellung gleich großen Zeitabschnitten, beispielsweise 1 Stunde, entspricht.

Eine besonders vorteilhafte Gestaltung der Anzeige der ersten bzw. der zweiten Zeitspanne ist dadurch gegeben, dass jedes des mindestens einen ersten Anzeigeelements ein Segment auf mindestens einem ersten Kreis bildet und/oder dass jedes des mindestens einen zweiten Anzeigeelements ein Segment auf mindestens einem zweiten Kreis bildet. Somit ist wie auf einer eine analoge Zeitdarstellung wiedergebenden Uhr der Zeitfortschritt der jeweiligen Zeitspannen auf den ersten Blick erkennbar.

Die leichte und schnelle Erkennbarkeit wird noch dadurch verbessert, dass sich der mindestens eine erste Kreis für das mindestens eine erste Anzeigeelement und der mindestens eine zweite Kreis für das mindestens eine zweite Anzeigeelement zueinander konzentrisch sind. Wenn beispielsweise das zweite Visualisierungsmittel Anzeigeelemente auf einem äußeren Kreis aufweist und das erste Visualisierungsmittel Anzeigeelemente auf einem oder mehreren inneren Kreisen, werden die die Kreise bildenden Anzeigeelemente die Kreise mit fortschreitender Zeit von außen nach innen nach und nach auffüllen. Alternativ können die zweiten Anzeigeelemente auch zusammen mit ersten Anzeigeelementen auf einem ersten äußeren Kreis und weitere zweite Anzeigeelemente auf weiteren weiter innen angeordneten Kreisen angeordnet sein.

Beispielsweise kann jeder der ersten und zweiten Kreise jeweils einen Zeitraum von 1 Tag symbolisieren. Falls die Kreise durch Kreissegmente zusammengesetzt sind, kann jedes der Kreissegmente 1 Stunde symbolisieren, so dass sich jeweils 24 Kreissegmente auf einem Kreis befinden. Selbstverständlich sind auch andere Einteilungen der Zeiträume für die Kreise und für die einzelnen Kreissegmente möglich. Die Anzeigeelemente können im Wesentlichen rechteckig sein oder auch eine beliebige andere Form annehmen. Beispielsweise können sie kreisförmig oder sternförmig sein. Alternativ können anstelle von Kreissegmenten natürlich auch andere Anzeigedarstellungen verwendet werden, beispielsweise schrittweise oder kontinuierlich anschwellende Balken oder Zeiger auf einer Skala oder auch eine völlig andere Darstellung, die den Zeitfortschritt auf einen Blick erkennen lässt. Eine Ziffernanzeige zeigt den Zeitfortschritt durch eine Zeitangabe numerisch an.

In einer besonders vorteilhaften Ausführungsform der Erfindung symbolisieren die ersten Anzeigeelemente des ersten Visualisierungsmittels und die zweiten Anzeigeelemente des zweiten Visualisierungsmittels jeweils Zeitabschnitte, die zurückgelegt worden sind oder die noch zurückzulegen sind. Wie in der oben angegebenen Ausführungsform können diese ersten und zweiten Anzeigeelemente in Form von Kreissegmenten auf einem oder mehreren Kreisen angeordnet sein. Für das erste Visualisierungsmittel können in diesem Falle Kreissegmente verwendet werden, die flächig dargestellt sind, und für das zweite Visualisierungsmittel Kreissegmente, die lediglich Rahmen darstellen. Besonders vorteilhaft ist es, wenn die zweiten Anzeigeelemente des zweiten Visualisierungsmittels in Form einer Umrahmung für die flächigen Darstellungen der ersten Anzeigeelemente des ersten Visualisierungsmittels wiedergegeben sind. Somit befinden sich die zweiten Anzeigeelemente des zweiten Visualisierungsmittels in diesem Falle auf demselben Kreis wie die ersten Anzeigeelemente des ersten Visualisierungsmittels. Allerdings können, wie zuvor bereits beschrieben, natürlich noch weitere erste Anzeigeelemente des ersten Visualisierungsmittels vorgesehen sein, die sich auf weiteren Kreisen befinden, auf denen sich keine zweiten Anzeigeelemente des zweiten Visualisierungsmittels befinden. Beispielsweise können die ersten Anzeigeelemente und die zweiten Anzeigeelemente gemeinsam einen äußere Kreis bilden und die weiteren ersten Anzeigeelemente einen weiteren inneren Kreis. Wie bereits dargestellt, können zumindest teilweise auch zweite Anzeigeelemente auf dem nach innen versetzten Kreis möglich. Grundsätzlich sind auch weitere beispielsweise noch weiter innen liegende Kreise für Anzeigeelemente denkbar.

In einer weiteren vorteilhaften Ausgestaltung der zuletzt beschriebenen Ausführungsform der Erfindung zeigen die zweiten Anzeigeelemente des zweiten Visualisierungsmittels den Zeitfortschritt seit dem zweiten Referenz-Einnahmezeitpunkt durch sukzessives Ausschalten von zunächst angezeigten zweiten Anzeigeelementen an. D.h. bei einer Entnahme einer Arzneimittelportion und somit bei Festlegung eines zweiten Referenz-Einnahmezeitpunktes werden zunächst alle zweiten Anzeigeelemente angezeigt, d.h. es werden die Anzeigeelemente für Zeitabschnitte bis zum nächsten (noch nicht erreichten) zweiten Referenz-Einnahmezeitpunkt dargestellt. Falls der zweite Referenz-Einnahmezeitpunkt bei der Entnahme der Arzneimittelportion allerdings bereits zurückliegt, werden bei einer Entnahme einer Arzneimittelportion nicht sämtliche zweiten Anzeigeelemente des zweiten Visualisierungsmittels angezeigt, sondern nur noch diejenigen, die der noch verbleibenden Zeitspanne bis zum nächsten Referenz-Einnahmezeitpunkt entsprechen. Die ersten Anzeigeelemente des ersten Visualisierungsmittels können in diesem Falle wie im zuvor beschriebenen Fall mit der anwachsenden Zeitüberschreitung sukzessive angezeigt werden. Somit zeigt das zweite Visualisierungsmittel nach einer Entnahme einer Arzneimittelportion unmittelbar zunächst die noch verbleibende Zeitspanne bis zum nächsten Referenz-Einnahmezeitpunkt an, während das erste Visualisierungsmittel bei einer sich anschließenden Zeitüberschreitung unmittelbar die bereits angefallene Zeitüberschreitung anzeigt.

Ferner kann das erste Visualisierungsmittel eine erste Zone und eine von der ersten Zone räumlich abgegrenzte zweite Zone umfassen, wobei die zweite Zone dazu dient, eine Überschreitung eines vorgegebenen dritten Zeitpunktes darzustellen. Wenn die Überschreitung des ersten Referenz-Einnahmezeitpunktes um eine vorgegebene dritte Zeitspanne stattfindet, ohne dass der Nutzer / die Nutzerin eine Arzneimittelportion nimmt, können sich ernsthafte Konsequenzen aus der fehlerhaften Einnahme ergeben. Dies wird dann durch eine beispielsweise in einer anderen (Signal)Farbe gehaltenen Darstellung von ersten Anzeigeelementen in der von der ersten Zone räumlich abgegrenzt gehaltenen zweiten Zone signalisiert. Beispielsweise können die Anzeigeelemente in der ersten und der zweiten Zone jeweils einen Zeitraum von einem Tag darstellen, so dass insgesamt eine Überschreitung des ersten Referenz-Einnahmezeitpunktes von 2 Tagen dargestellt werden kann. Grundsätzlich ist natürlich auch eine Überschreitung des ersten Referenz-Einnahmezeitpunktes um einen kürzeren oder längeren Zeitraum möglich.

In einer weiter bevorzugten Ausführungsform der Erfindung ist zusätzlich eine Anzeige der Anzahl der sich in dem Spender befindenden Arzneimittelportionen vorgesehen. Die Anzeige kann beispielsweise eine digitale Ziffernanzeige sein. Dadurch wird dem Nutzer / der Nutzerin zusätzliche Sicherheit für die Einnahme der Arzneimittelportionen verschafft, weil somit sichergestellt wird, dass der Nutzer / die Nutzerin immer dafür sorgen kann, dass stets mindestens eine Arzneimittelportion zur Verfügung steht. Der Nutzer / die Nutzerin kann dadurch beispielsweise auch für eine bevorstehende Reise in ausreichender Weise disponieren.

In einer weiter bevorzugten Ausführungsform der Erfindung ist zusätzlich eine Anzeige der Anzahl der in einem Einnahmezyklus bereits genommenen Arzneimittelportionen vorgesehen. Diese Information kann ebenfalls durch eine Ziffernanzeige angezeigt werden. Somit ist es für den Nutzer / die Nutzerin möglich, den Verlauf der Einnahme von Arzneimittelportionen zu überwachen. Im Falle der Kontrazeption mit flexiblem Einnahmeregime hat es die Nutzerin damit in der Hand zu entscheiden, wann sie nach Ablauf einer Einnahmephase, in der sie 24 Kontrazeptiva-Portionen genommen hat (obligatorische Einnahmephase), eine Unterbrechungsphase einleitet. Dies ist im Hinblick auf die Beschränkungen bei der Entscheidung über die Einleitung der Unterbrechungsphase wichtig, da die Unterbrechungsphase nicht vor Anlauf der obligatorischen Einnahmephase eingeleitet werden darf und spätestens nach einer Einnahme von insgesamt beispielsweise 120 Kontrazeptiva-Portionen eingeleitet werden muss.

In einer weiter bevorzugten Ausführungsform der Erfindung ist zusätzlich eine Anzeige für eine Einnahmeunterbrechung vorgesehen. Dies kann durch ein hierfür geeignetes Symbol realisiert werden. Somit wird der Benutzerin von Kontrazeptiva angezeigt, dass eine Einnahmeunterbrechung eingeleitet wurde und dass daher aktuell keine Arzneimittelportion genommen werden darf. Die Unterbrechungsphase dauert in einem flexiblen Regime üblicherweise 4 Tage. Nach Ablauf der Unterbrechungsphase wird dann wieder eine Einnahmephase in einem neuen Einnahmezyklus eingeleitet. Hierzu entnimmt die Nutzerin eine erste Arzneimittelportion in diesem neuen Einnahmezyklus, triggert damit das zweite Visualisierungsmittel und legt somit auch die neuen Referenz-Einnahmezeitpunkte fest.

Selbstverständlich kann die Ansteuerung für die Anzeige auch eine Logik enthalten, wonach eine Einleitung der Unterbrechungsphase dann ausgeschlossen wird, wenn noch keine 24 Arzneimittelportionen im aktuellen Einnahmezyklus genommen worden sind (obligatorische Einnahmephase). Dies kann dann beispielsweise dadurch angezeigt werden, dass das die Einnahmeunterbrechung anzeigende Symbol nicht oder durchgestrichen erscheint. Außerdem kann beispielsweise ein optisch, akustisch und/oder haptisch wahrnehmbares Warnsignal angezeigt werden.

Abgesehen davon, dass die Nutzerin dann, wenn das Arzneimittel ein in einem flexiblen Regime einnehmbares Kontrazeptivum ist, von sich aus von der Einnahmephase in die Unterbrechungsphase übergehen kann, wenn sie sich nicht mehr in der obligatorischen Einnahmephase befindet, kann vorgesehen sein, dass die Anzeigeeinrichtung die Unterbrechungsphase automatisch einleitet, wenn die letzte Einnahme einer Arzneimittelportion eine Mindestzeitspanne zurückliegt. Beispielsweise kann vorgesehen sein, dass die Unterbrechungsphase automatisch eingeleitet wird, wenn der zweite Referenz-Einnahmezeitpunkt 72 Stunden zurückliegt. Durch Überwechseln in die Unterbrechungsphase kann in diesem Falle auf der Anzeige aktuell dargestellt werden, dass die Unterbrechungsphase bereits seit 48 Stunden andauert. Ein derartiger automatischer Übergang ist dann, wenn die Nutzerin die flexible Einnahmephase bereits erreicht hat, ohne weiteres möglich. Aber auch dann, wenn sich die Nutzerin noch in der obligatorischen Phase befindet, kann dieser automatische Übergang in einer möglichen Ausführungsform der Erfindung stattfinden. Eine sichere Kontrazeption ist in letzterem Falle zwar nicht mehr gegeben. Mit dem automatischen Übergang in die Unterbrechungsphase wird aber wieder ein ordnungsgemäßes Einnahmeregime angestrebt.

In einer weiter bevorzugten Ausführungsform der Erfindung schließt die Anzeige für die Einnahmeunterbrechung eine Anzeige der Anzahl der Tage der Einnahmeunterbrechung ein. Diese Anzeige kann wiederum in Form einer digitalen Ziffernanzeige gebildet sein.

In einer weiter bevorzugten Ausführungsform der Erfindung zeigen die ersten oder die zweiten Visualisierungsmittel während der Einnahmeunterbrechung die zwischen zwei Referenz-Einnahmezeitpunkten verstrichene Zeitspanne an. Bei diesen Referenz-Einnahmezeitpunkten handelt es sich um die Zeitpunkte, die sich wie der erste Referenz-Einnahmezeitpunkt und der zweite Referenz-Einnahmezeitpunkt aus dem Zeitpunkt der ersten Entnahme einer Arzneimittelportion in einem Einnahmezyklus berechnen und die im Zeitraum der Einnahmeunterbrechung liegen. Somit kann der Zeitfortschritt auch während einer Einnahmeunterbrechung verfolgt werden, so dass die Nutzerin in dieser Phase fortwährend einen guten Überblick auch über den Status der Unterbrechungsphase hat.

In einer bevorzugten Ausführungsform kann ferner vorgesehen sein, dass dem Nutzer / der Nutzerin angezeigt wird, dass sie zwei Arzneimittelportionen nehmen muss, wenn der zweite Referenz-Einnahmezeitpunkt länger als 2 Einnahmeperioden, vorzugsweise aber nicht länger als 3 Einnahmeperioden, zurückliegt, d.h. der Nutzer / die Nutzerin entnimmt in diesem Falle alle (beiden) bis dahin versäumten Arzneimittelportionen. Um den Nutzer in einem solchen Falle zu veranlassen, alle versäumten Arzneimittelportionen zu nehmen, kann beispielsweise vorgesehen sein, dass die Anzeige der Zeitüberschreitung (durch das erste Visualisierungsmittel) bei einer Entnahme nur einer Arzneimittelportion nur im Umfange des Zeitraumes gelöscht wird, der der Entnahme einer einzelnen Arzneimittelportion entspricht, bei täglicher Einnahme einer Arzneimittelportion also im Umfange des Zeitraumes, der einem Tag entspricht. Somit verbleibt bei einer Entnahme von zunächst nur einer Arzneimittelportion immer noch eine Anzeige einer Restzeitüberschreitung, die allerdings nach der Entnahme dieser Arzneimittelportion nun um 24 Stunden geringer ausfällt. Erst wenn dann auch eine zweite Arzneimittelportion genommen worden ist, werden die Anzeige der Zeitüberschreitung vollständig und zumindest zum Teil auch die Anzeige durch das zweite Visualisierungsmittel gelöscht, das den Zeitraum seit dem zweiten Referenz-Einnahmezeitpunkt anzeigt.

Handelt es sich bei den Arzneimitteln um Kontrazeptiva, die in einem flexiblen Einnahmeregime genommen werden, kann eine derartige Vorgehensweise grundsätzlich sowohl für den Fall vorgesehen sein, dass sich die Nutzerin in der obligatorischen Phase oder in der flexiblen Phase befindet. Bei einer Zeitüberschreitung von bis zu 48 Stunden (d.h. einem Zeitraum von bis zu 72 Stunden seit dem zweiten Referenz-Einnahmezeitpunkt) findet dann vorzugsweise noch kein automatischer Übergang von der Einnahmephase in die Unterbrechungsphase statt. Falls die Zeitspanne seit dem zweiten Referenz-Einnahmezeitpunkt jedoch 72 Stunden oder mehr beträgt, d.h. dass 3 oder mehr Arzneimittelportionen genommen werden sollten und nicht genommen worden sind, nimmt die Anzeige einen automatischen Übergang in die Unterbrechungsphase vor. Falls sich die Nutzerin in diesem Falle in der obligatorischen Phase befindet, wird vorzugsweise zusätzlich angezeigt, dass außerdem weitere Verhütungsmittel verwendet werden müssen (backup contraception), da eine wirksame Kontrazeption unter diesen Bedingungen nicht mehr gegeben ist. Bei einem solchen Vorfall kann vorgesehen sein, dass die Nutzerin mit einem optischen und/oder akustischen und/oder haptischen Alarmsignal alarmiert wird und zusätzlich vorzugsweise optisch der Hinweis gegeben wird, zusätzlich zur weiteren Einnahme der Arzneimittelportionen ein zusätzliches Verhütungsmittel anzuwenden.

Zusätzliche kontrazeptive Maßnahmen sind in jedem Falle dann erforderlich, wenn eine sichere Kontrazeption nicht mehr gewährleistet ist. Eine sichere Kontrazeption ist nur unter folgender Bedingung garantiert:
Bei fehlender Einnahme von Arzneimittelportionen an einem einzigen Tag oder an mehreren Tagen innerhalb eines Zeitraumes von maximal 7 Tagen mit unregelmäßiger Einnahme, der dadurch gekennzeichnet ist, dass vor dem ersten Tag dieses Zeitraumes, an dem keine Arzneimittelportion genommen wurde, während mindestens 7 Tagen ununterbrochen Arzneimittelportionen eingenommen wurden, schließt sich an den letzten Tag dieses Zeitraumes, an dem keine Arzneimittelportion genommen wurde, wiederum eine mindestens 7-tägige Zeitspanne an, in der ununterbrochen Arzneimittelportionen genommen werden.

Anders ausgedrückt darf eine Phase mit unregelmäßiger Einnahme an mehreren Tagen zwischen zwei Phasen mit jeweils mindestens 7-tägiger regelmäßiger Einnahme nicht länger als 7 Tage andauern, um eine sichere Kontrazeption zu erreichen. Andernfalls müssen zusätzliche kontrazeptive Maßnahmen angewendet werden.

Es hat sich gezeigt, dass das Absinken des Plasmaspiegels der Wirkstoffe des Kontrazeptivums in diesen Fällen nicht zu einer Gefährdung des kontrazeptiven Schutzes führt.

Falls die vorgenannten Bedingungen dagegen nicht erfüllt sind, werden zusätzliche kontrazeptive Maßnahmen erforderlich. Der erste Tag, an dem zusätzliche kontrazeptive Maßnahmen erforderlich werden, ist ein Tag mit Fehleinnahme, der auf den vorgenannten 7-tägigen Zeitraum mit unregelmäßiger Einnahme innerhalb von weniger als 7 Tagen mit ununterbrochener Einnahme von Arzneimittelportionen folgt. In diesem Moment ergibt sich, gerechnet vom ersten Tag mit Fehleinnahme bis zum letzten Tag mit Fehleinnahme ein Fehleinnahme-Zeitraum von mehr als 7 Tagen, ohne dass dieser Fehleinnahme-Zeitraum durch einen Zeitraum von 7 Tagen ununterbrochener Einnahme von Arzneimittelportionen unterbrochen wäre.

Anders ausgedrückt muss nach einer Phase mit unregelmäßiger Einnahme an bis zu 7 aufeinander folgenden Tagen eine Phase von mindestens 7 Tagen mit ununterbrochener Einnahme von Arzneimittelportionen folgen, um die kontrazeptive Wirkung aufrechtzuerhalten.

Das Erfordernis zusätzlicher kontrazeptiver Maßnahmen dauert dann so lange an, bis wieder ein Zeitraum von mindestens 7 Tagen währender ununterbrochener Einnahme abgelaufen ist.

Die vorgenannten Regeln sind davon unabhängig, ob Tabletten an Tagen nicht genommen werden, an denen gemäß dem vorgesehenen Einnahmeregime Tabletten zu nehmen sind (Einnahmephase) oder ob diese Tage in eine Unterbrechungsphase fallen, d.h. dass an diesen Tagen gemäß dem Einnahmeregime keine Tabletten zu nehmen sind. Anders ausgedrückt werden Tage, an denen gemäß dem Einnahmeregime keine Tabletten zu nehmen sind, wie Tage mit Fehleinnahme behandelt.

Die Anzeigeeinrichtung wird vorzugsweise derart ausgelegt, dass sie unter den vorstehend genannten Bedingungen an jedem Tag anzeigt, ob zusätzliche kontrazeptive Maßnahmen erforderlich sind. Hierzu kann automatisch ein Warnsymbol wegen fehlender Kontrazeption, beispielsweise ein Ausrufezeichen ("!"), auf der Anzeige erscheinen. Gemäß den vorstehenden Erläuterungen erscheint dieses Warnsymbol unter folgenden Bedingungen:
a) Es gibt einen Fehleinnahme-Zeitraum, der dadurch gekennzeichnet ist,
   i) dass sich der Fehleinnahme-Zeitraum an einen ersten Einnahme-Zeitraum von mindestens 7 Tagen mit ununterbrochener Einnahme von Arzneimittelportionen anschließt,
   ii) dass sich an den Fehleinnahme-Zeitraum wiederum ein zweiter Einnahme-Zeitraum von mindestens 7 Tagen mit geforderter ununterbrochener Einnahme von Arzneimittelportionen anschließt,
   iii) wobei an mindestens einem Tag in dem Fehleinnahme-Zeitraum keine Arzneimittelportion genommen wird,
   iv) dass innerhalb des Fehleinnahme-Zeitraumes kein Zeitraum mit mindestens 7-tägiger ununterbrochener Einnahme eingeschlossen ist,
   v) dass an dem ersten und letzten Tag des Fehleinnahme-Zeitraumes keine Arzneimittelportion genommen worden ist und
   vi) dass der Fehleinnahme-Zeitraum länger als 7 Tage andauert;
b) der erste Tag, an dem das Warnsymbol erscheint, ist der 8. Tag des Fehleinnahme-Zeitraumes;
c) der letzte Tag, an dem das Warnsymbol erscheint, ist der 7. Tag des sich an den Fehleinnahme-Zeitraum anschließenden zweiten Einnahme-Zeitraumes mit ununterbrochener Einnahme von Arzneimittelportionen.

In einer weiter bevorzugten Ausführungsform der Erfindung ist die Anzeigeeinrichtung eine digitale Anzeigeeinrichtung. Insbesondere kann die Anzeigeeinrichtung eine LCD-Anzeigeeinrichtung sein. Beispielsweise kann eine OLED-Anzeige vorgesehen sein. Alternativ können auch beliebige andere Technologien zum Einsatz kommen.

Weiterhin kann die Anzeigeeinrichtung auch weitere Anzeigeelemente für andere Funktionen enthalten, beispielsweise ein Symbol für einen niedrigen Batterieladungszustand, ein Symbol für die Anzeige, ob ein akustisches Wamsignal eingeschaltet oder ausgeschaltet ist, ein Symbol zum Hinweis an den Nutzer / die Nutzerin, die Bedienungsanleitung zu Rate zu ziehen, ein Symbol für den Hinweis, dass die digitale Ziffernanzeige die Anzahl der sich noch im Spender befindenden Arzneimittelportionen anzeigt, ein Symbol für den Hinweis, dass ein niedriger Stand an Arzneimittelportionen vorliegt, ein Symbol für den Hinweis, dass die digitale Ziffernanzeige die Anzahl der in einem Einnahmezyklus bereits genommenen Arzneimittelportionen anzeigt, ein Symbol zur Bestätigung, dass eine Arzneimittelportion genommen wurde, sowie ein Symbol als Warnhinweis, dass eine Unterbrechungsphase bei einem entsprechenden Versuch der Nutzerin nicht eingeleitet werden darf. Diese Symbole können jeweils als Daueranzeige oder in Form einer blinkenden Anzeige dargestellt sein. Die Symbole werden vorzugsweise alternativ oder teilweise auch gleichzeitig auf der Anzeigeeinrichtung angezeigt. Außerdem kann die Anzeigeeinrichtung so ausgebildet sein, dass weitere optisch, akustisch und/oder haptisch wahrnehmbare Warnsignale abgegeben werden können.

Die Anzeigeeinrichtung umfasst eine elektronische Ansteuerung für die Anzeige. Dies kann in üblicher Art und Weise eine programmierte oder programmierbare Mikroprozessorschaltung sein.

Die Anzeigeeinrichtung wird vorzugsweise durch Bedienelemente am Spender bedient. Dies können insbesondere Taster oder andere Sensoren sein. Beispielsweise können ein Menütaster sowie ein Bestätigungstaster vorgesehen sein. Der Menütaster dient zur Auswahl bestimmter Anzeige- oder Auswahlmodi. Der Bestätigungstaster dient dazu, in einem bestimmten Modus eine mögliche Auswahl zu treffen.

Der Spender kann zusätzlich zu der Anzeige weitere optische Anzeigeelemente, wie LEDs sowie zusätzlich akustische Alarmeinrichtungen (Lautsprecher) und/oder mechanische Alarmeinrichtungen (Vibratoren) umfassen.

Alle Anzeigeelemente der Anzeigeeinrichtung können entweder ständig angezeigt oder blinkend angezeigt werden. Außerdem können die Anzeigeelemente auch entweder in einer neutralen (möglichst kontrastreichen) Farbe dargestellt sein, beispielsweise in schwarzer Farbe, oder alle oder auch nur einzelne Anzeigeelemente können in einer Signalfarbe ausgeführt sein, beispielsweise in roter Farbe. Um die Bedeutung der Anzeige bestimmter Bedienungszustände im Spender und in der Anzeigeeinrichtung zu betonen, können Eskalationsstufen vorgesehen sein, beispielsweise anstelle einer schwarzen Darstellung eine farbige, beispielsweise rote, Darstellung. Alternativ oder kumulativ kann anstelle einer Dauerdarstellung eine blinkende Darstellung gewählt werden.

Besonders bevorzugt ist es, wenn die Anzeigeeinrichtung mit einem Spender für die Arzneimittelportionen kombiniert ist. In diesem Falle kann der Spender die Anzeigeeinrichtung im Falle der Entnahme einer Arzneimittelportion ohne eine Zwischenschaltung des Nutzers / der Nutzerin triggern, d.h. eine Entnahme einer Arzneimittelportion führt unmittelbar zu einer Berücksichtigung einer Entnahme einer Arzneimittelportion, ohne dass der Nutzer / die Nutzerin hierzu eine separate Eingabe für die Entnahme machen muss. Diese Triggerung kann mechanisch, elektromechanisch, elektronisch oder auf andere Art und Weise stattfinden. Insbesondere kann der Spender eine auf mechanischer oder elektromechanischer Kraftübertragung von Betätigungsmitteln zu einer Ausgabeeinheit beruhende Ausgabe von Arzneimittelportionen ermöglichen, und es kann vorgesehen sein, dass die mechanische oder elektromechanischer Kraftübertragung bei der Entnahme einer Arzneimittelportion unmittelbar auf die Anzeigeeinrichtung wirkt, beispielsweise mittels eines Schalters, so dass damit die Entnahme in der Anzeigeeinrichtung ohne zusätzliche Manipulationen des Nutzers / der Nutzerin registriert wird. Durch die automatische Triggerung wird einer Fehlbedienung weitgehend dadurch vorgebeugt, dass der Nutzer / die Nutzerin die Entnahme in der Anzeigeeinrichtung nicht falsch verbuchen kann.

Die Arzneimittelportionen können wie bei herkömmlichen Spendern in Form von Tabletten, Pillen, Dragees o.ä. zubereitet sein. Die Portionen können grundsätzlich in Form von Blistern abgepackt sein. Bevorzugt ist eine Abpackung der Arzneimittelportionen in Kartuschen, d.h. in säulenförmiger Anordnung. Die Arzneimittelportionen können in diesem Falle durch eine axiale Öffnung in der Kartusche ausgegeben werden.

Die vorliegende Erfindung wird an Hand der nachfolgend beschriebenen Figuren näher dargestellt. Die darin gezeigten Ausführungsformen sowie die Beschreibung der Figuren dienen lediglich zur Veranschaulichung der Erfindung, nicht jedoch zu deren Beschränkung.
- Fig. 1: zeigt einen Spender mit einer erfindungsgemäßen Anzeigeeinrichtung in einer Vorderansicht;
- Fig. 2: zeigt den Spender in einer Rückansicht;
- Fig. 3: zeigt die Anzeige der erfindungsgemäßen Anzeigeeinrichtung in einer ersten erfindungsgemäßen Ausführungsform in der Draufsicht;
- Fig. 4: zeigt die Anzeige der erfindungsgemäßen Anzeigeeinrichtung in der ersten erfindungsgemäßen Ausführungsform vor dem ersten Einschieben einer Kartusche und danach in unterschiedlichen Anzeigemodi;
- Fig. 5: zeigt die Anzeige der erfindungsgemäßen Anzeigeeinrichtung in der ersten erfindungsgemäßen Ausführungsform nach der ersten Entnahme einer Tablette in einem Einnahmezyklus;
- Fig. 6: zeigt die Anzeige der erfindungsgemäßen Anzeigeeinrichtung in der ersten erfindungsgemäßen Ausführungsform vor und nach einer vor Ablauf des zweiten Referenz-Einnahmezeitpunktes stattfindenden Entnahme einer Tablette;
- Fig. 7: zeigt die Anzeige der erfindungsgemäßen Anzeigeeinrichtung in der ersten erfindungsgemäßen Ausführungsform vor und nach einer vor Beginn des Einnahmezeitintervalls stattfindenden Entnahme einer Tablette;
- Fig. 8a: zeigt die Anzeige der erfindungsgemäßen Anzeigeeinrichtung in der ersten erfindungsgemäßen Ausführungsform nach Überschreiten des ersten Referenz-Einnahmezeitpunktes und zusätzlich mit Umschaltung (Ein-/ Ausschalten) der akustischen Warnfunktion;
- Fig. 8b, 8c: zeigt die Anzeige der erfindungsgemäßen Anzeigeeinrichtung in der ersten erfindungsgemäßen Ausführungsform nach Überschreiten des ersten Referenz-Einnahmezeitpunktes bei unterschiedlichen ersten Zeitspannen;
- Fig. 9: zeigt die Anzeige der erfindungsgemäßen Anzeigeeinrichtung in der ersten erfindungsgemäßen Ausführungsform beim Übergang in die Unterbrechungsphase;
- Fig. 10: zeigt die Anzeige der erfindungsgemäßen Anzeigeeinrichtung in der ersten erfindungsgemäßen Ausführungsform während der Unterbrechungsphase;
- Fig. 11a: zeigt die Anzeige einer erfindungsgemäßen Anzeigeeinrichtung in der ersten erfindungsgemäßen Ausführungsform vor und nach einer Entnahme von zwei Tabletten ohne Berücksichtigung der zweiten Tablette für die Einnahme nach kurzer Überschreitung des ersten Referenz-Einnahmezeitpunktes;

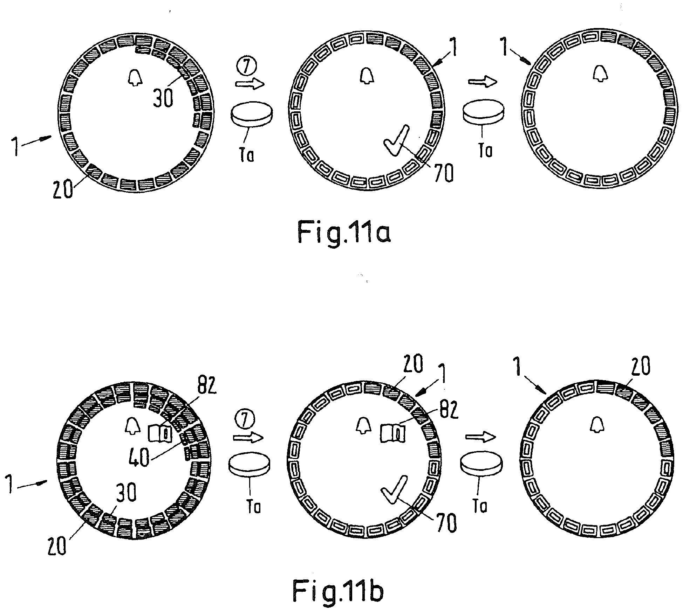

- Fig. 11 b:: zeigt die Anzeige der erfindungsgemäßen Anzeigeeinrichtung in der ersten erfindungsgemäßen Ausführungsform vor und nach einer Entnahme von zwei Tabletten ohne Berücksichtigung der zweiten Tablette für die Einnahme nach längerer Überschreitung des ersten Referenz-Einnahmezeitpunktes;

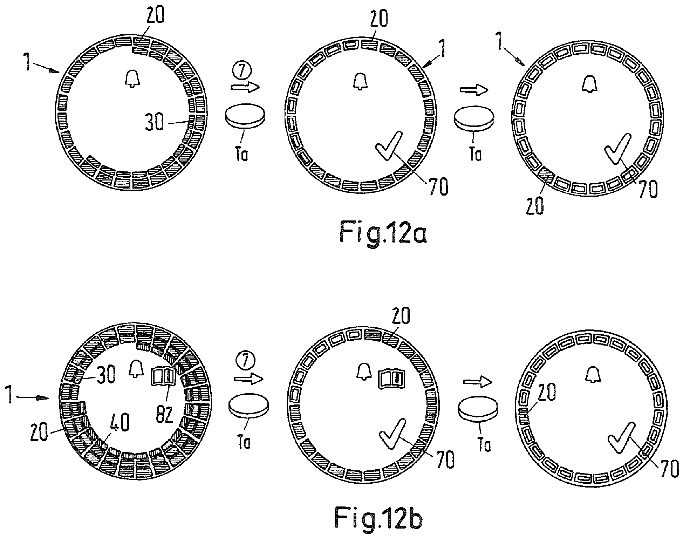

- Fig. 12a: zeigt die Anzeige der erfindungsgemäßen Anzeigeeinrichtung in der ersten erfindungsgemäßen Ausführungsform vor und nach einer Entnahme von zwei Tabletten mit Berücksichtigung auch der zweiten Tablette für die Einnahme nach kurzer Überschreitung des ersten Referenz-Einnahmezeitpunktes;
- Fig. 12b:: zeigt die Anzeige der erfindungsgemäßen Anzeigeeinrichtung in der ersten erfindungsgemäßen Ausführungsform vor und nach einer Entnahme von zwei Tabletten mit Berücksichtigung auch der zweiten Tablette für die Einnahme nach längerer Überschreitung des ersten Referenz-Einnahmezeitpunktes;

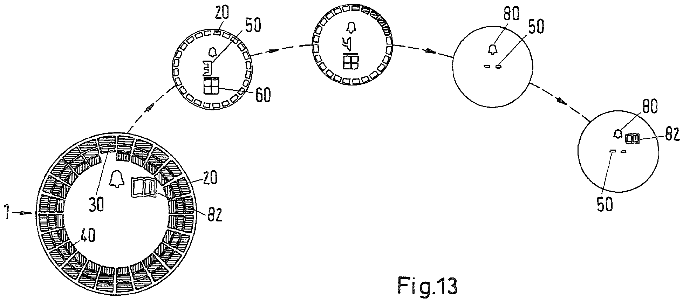

- Fig. 13: zeigt die Anzeige der erfindungsgemäßen Anzeigeeinrichtung in der ersten erfindungsgemäßen Ausführungsform beim automatischen Übergang von der flexiblen Phase in die Unterbrechungsphase;

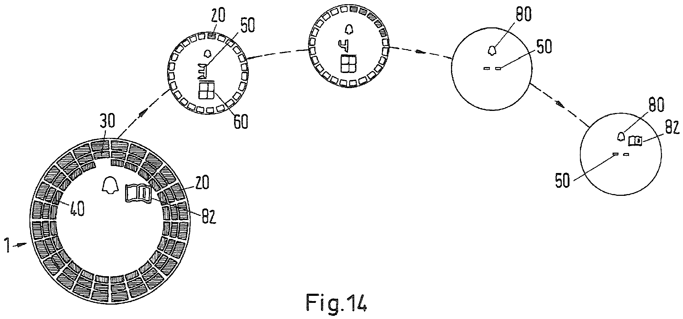

- Fig. 14: zeigt die Anzeige der erfindungsgemäßen Anzeigeeinrichtung in der ersten erfindungsgemäßen Ausführungsform beim automatischen Übergang von der obligatorischen Phase in die Unterbrechungsphase;

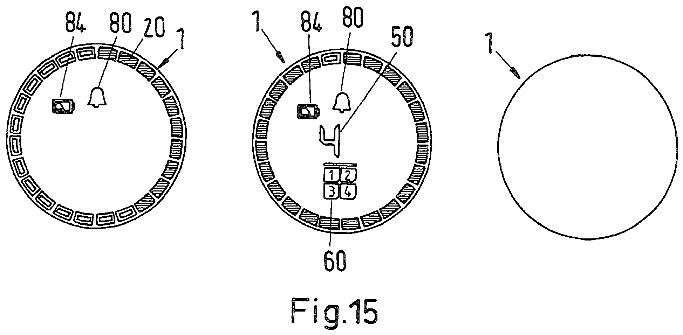

- Fig. 15: zeigt die Anzeige der erfindungsgemäßen Anzeigeeinrichtung in der ersten erfindungsgemäßen Ausführungsform bei niedrigem Batterieladezustand;

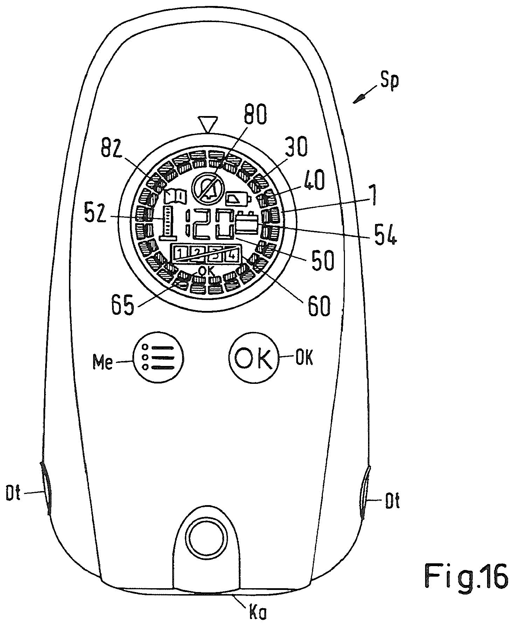

- Fig. 16: zeigt die Anzeige der erfindungsgemäßen Anzeigeeinrichtung in einer zweiten erfindungsgemäßen Ausführungsform mit alternativen Symboldarstellungen;

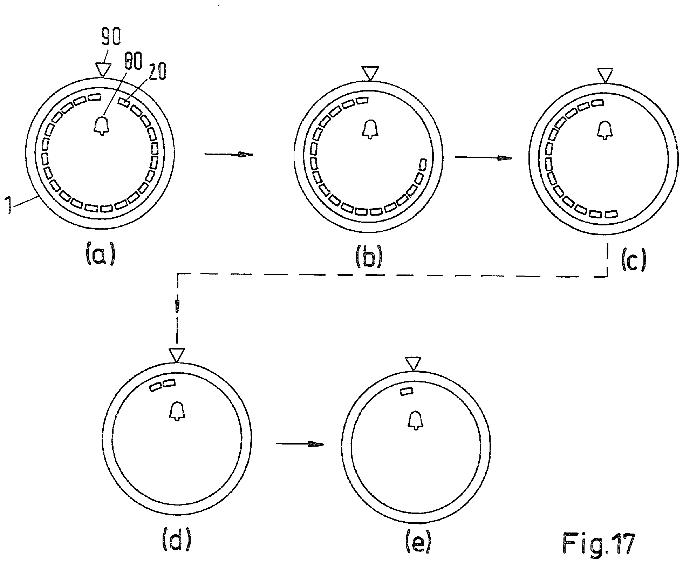

- Fig. 17: zeigt die Anzeige der erfindungsgemäßen Anzeigeeinrichtung in der zweiten erfindungsgemäßen Ausführungsform seit dem zweiten Referenz-Einnahmezeitpunkt in verschiedenen Stadien des Zeitfortschritts;

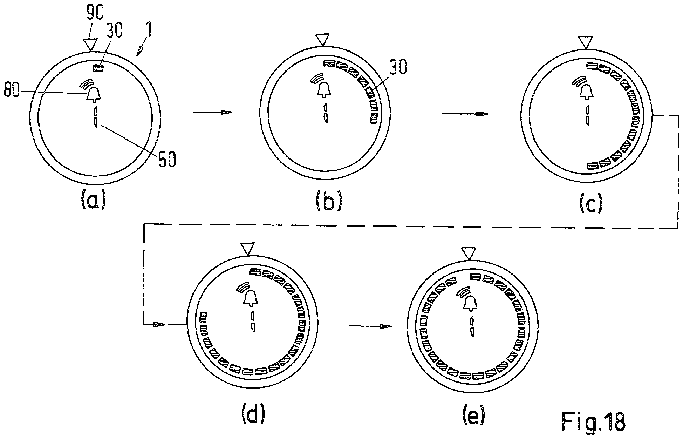

- Fig. 18: zeigt die Anzeige der erfindungsgemäßen Anzeigeeinrichtung in der zweiten erfindungsgemäßen Ausführungsform seit dem ersten Referenz-Einnahmezeitpunkt in verschiedenen Stadien des Zeitfortschritts;

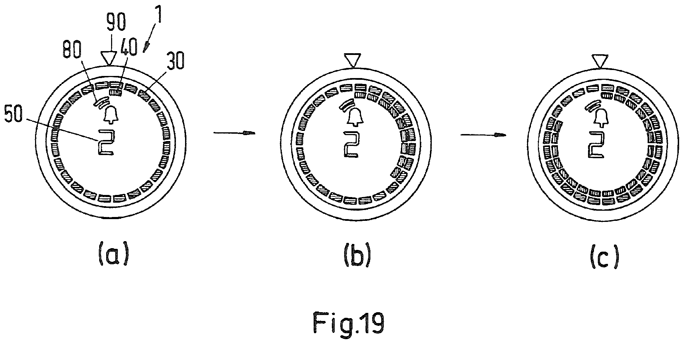

- Fig. 19: zeigt die Anzeige der erfindungsgemäßen Anzeigeeinrichtung in der zweiten erfindungsgemäßen Ausführungsform bei Zeitüberschreitung von mehr als einer Einnahmeperiode in verschiedenen Stadien des Zeitfortschritts;

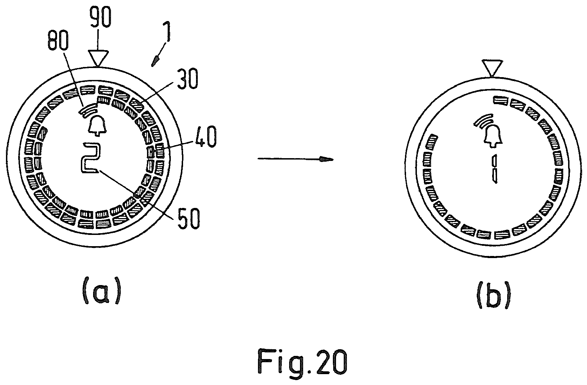

- Fig. 20: zeigt die Anzeige der erfindungsgemäßen Anzeigeeinrichtung in der zweiten erfindungsgemäßen Ausführungsform vor und nach der Entnahme einer Tablette;

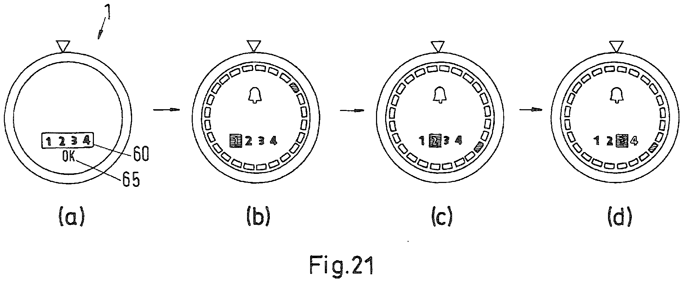

- Fig. 21: zeigt die Anzeige der erfindungsgemäßen Anzeigeeinrichtung in der zweiten erfindungsgemäßen Ausführungsform nach dem Einleiten einer Unterbrechungsphase;

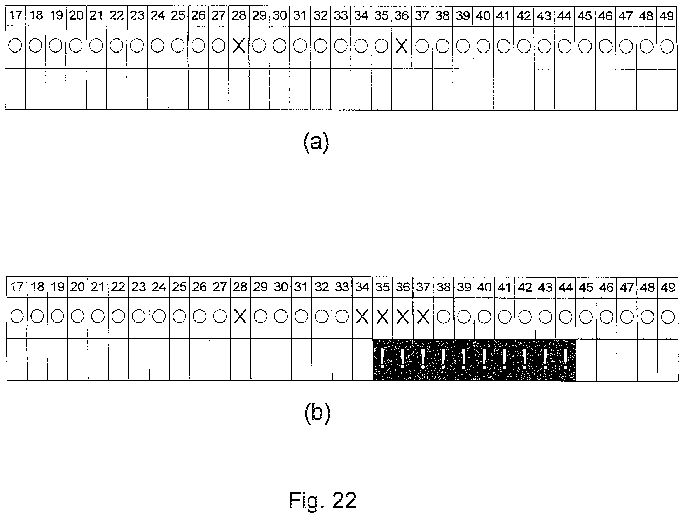

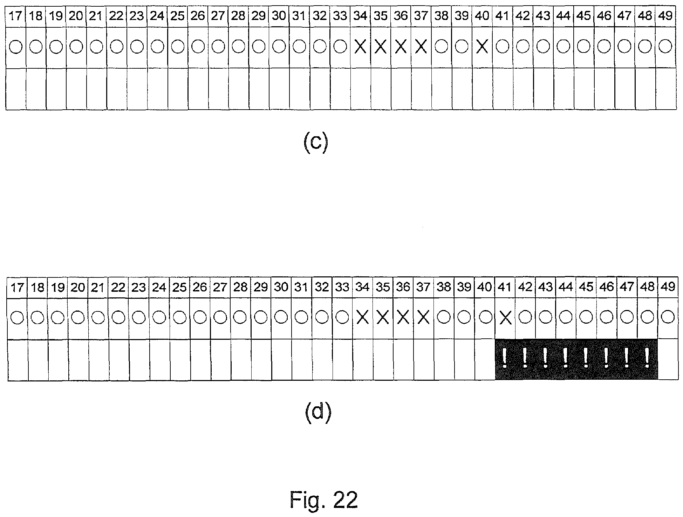

- Fig. 22: zeigt Beispiele für die Einnahme bzw. Fehleinnahme (= keine Einnahme) von Tabletten innerhalb eines Einnahmeregimes für die Kontrazeption.

Gleiche Elemente sind mit gleichen Bezugszeichen versehen. Soweit nachfolgend Tabletten erwähnt sind, geschieht dies zur vereinfachten Bezeichnung von Arzneimittelportionen. Daher sollen damit gleichfalls Pillen, Dragees, Kapseln und andere portionierte feste Darreichungsformen erfasst sein. Die Anzeigeeinrichtung wird nachfolgend für eine Anwendung für Arzneimittelportionen gezeigt und beschrieben, die Kontrazeptiva sind und die in einem flexiblen Einnahmeregime genommen werden, d.h. mit einer obligatorischen Einnahmephase von 24 Tagen, einer darauf folgenden flexiblen Einnahmephase von bis zu weiteren 96 Tagen (insgesamt maximal 120 Tage) und einer sich daran anschließenden Unterbrechungsphase von 4 Tagen.

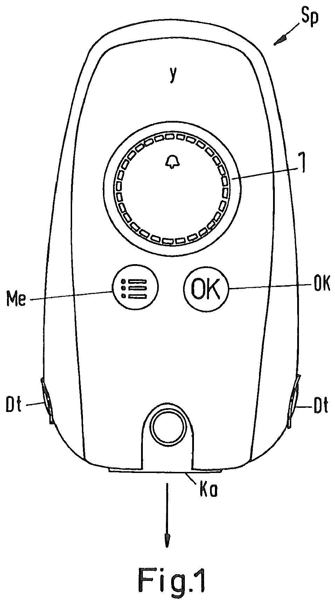

Fig. 1 zeigt einen Spender Sp für Arzneimittel in einer Ansicht von der Vorderseite. Im unteren Bereich des Spenders ist das Unterteil einer in den Spender von unten axial eingeschobenen Arzneimittel-Kartusche Ka sichtbar. Die Arzneimittelportionen werden nach unten ausgegeben (Pfeil). Hierzu weist die Kartusche an der Unterseite einen Auswurfmechanismus auf, der über seitliche Drucktaster Dt betätigt wird.

Weiterhin sind auf der Vorderseite des Spenders Sp eine Anzeigeeinrichtung mit einer Anzeige 1 sowie Bedienungstaster Me, Ok angebracht. Die Menütaste Me dient zur Auswahl eines bestimmten Anzeige- bzw. Auswahlmodus auf der Anzeige, und die Bestätigungstaste Ok dient zur Bestätigung der Auswahl einer Funktion, die von der Anzeige angezeigt ist.

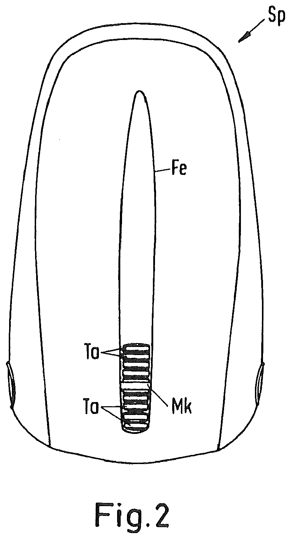

Fig. 2 zeigt den Spender Sp in einer Rückansicht. Das Gehäuse des Spenders Sp ist auf der Rückseite mittig axial mit einem Fenster Fe versehen, durch das ein Teil der sich in der Kartusche befindlichen Tabletten Ta sichtbar ist. Außerdem ist eine Marke Mk in Form einer Linie angebracht, die anzeigt, auf welcher Füllhöhe sich in der Kartusche noch gerade 7 Tabletten befinden. Somit hat die Nutzerin auch direkt eine optische Kontrolle über den Füllstand des Spenders mit Tabletten.

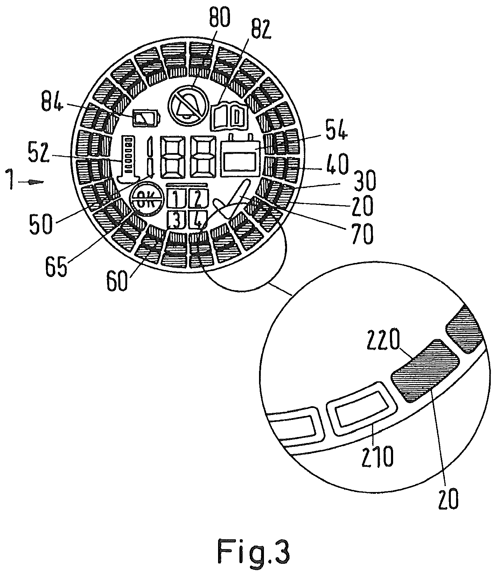

Fig. 3 zeigt die Anzeige 1 einer erfindungsgemäßen Anzeigeeinrichtung mit einer im Ausschnitt vergrößerten Darstellung von sich auf einem Kreis befindenden Anzeigeelementen 20. Diese Darstellung dient lediglich zur Veranschaulichung aller möglichen Anzeigeelemente und Symbole auf der Anzeige. Üblicherweise werden nicht alle Elemente und Symbole gleichzeitig sichtbar sein sondern lediglich nach Maßgabe der Anzeigesituation angezeigt.

Die Anzeige ist vorzugsweise eine LCD-Anzeige, die besonders vorzugsweise eine Beleuchtung oder zumindest optional eine Beleuchtung aufweist. Die hier dargestellten Anzeigeelemente und Symbole brauchen auch nicht fortwährend angezeigt zu werden: Während einer Ruhepause, d.h. während einer Phase, in der die Nutzerin den Spender nicht bedient und die Anzeigeeinrichtung nicht durch einen beliebigen Tastendruck aktiviert wird, kann die Anzeigeeinrichtung automatisch in eine Ruhestellung überführt werden, in der die Anzeigeelemente und Symbole nicht sichtbar sind. Die Anzeigeeinrichtung kann beispielsweise durch Tastendruck wieder in einen aktiven Zustand übergehen.

Die meisten Anzeigeelemente und Symbole der Anzeige 1 sind beispielsweise in schwarzer Farbe gehalten, um einen guten Kontrast zum Hintergrund zu erreichen. Einige Anzeigeelemente und Symbole können jedoch andersfarbig ausgestaltet sein.

Die Anzeige 1 ist mit sektoriellen Anzeigeelementen 20, 30, 40 ausgestattet, die sich auf jeweils einem Kreis befinden. Diese Kreise sind konzentrisch zueinander angeordnet. Die zweiten Anzeigeelemente 20 auf dem äußeren Kreis stellen zusammen genommen das zweite Visualisierungsmittel dar. 24 derartige sektorielle zweite Anzeigeelemente bilden einen Kreis. Im Falle des vorliegenden Ausführungsbeispiels sollen die 24 zweiten Anzeigeelemente jeweils 1 Stunde darstellen, so dass alle 24 zweiten Anzeigeelemente 24 Stunden, d.h. 1 Tag, symbolisieren. Zur Darstellung, wo sich die zweiten Anzeigeelemente befinden, sind Anzeigerahmen 210 vorgesehen, die auch dann sichtbar sind, wenn die in diesen Rahmen angeordneten Anzeigeflächen 220 der zweiten Anzeigeelemente nicht dargestellt sind. Die ersten Anzeigeelemente 30 und 40 stellen zusammen genommen das erste Visualisierungsmittel dar. Während die ersten Anzeigeelemente 30 in schwarzer Farbe gehalten sind, können die ersten Anzeigeelemente 40 in roter Farbe dargestellt sein. Jeweils 24 derartige sektorielle zweite Anzeigeelemente bilden einen Kreis, wobei zwei Kreise von zweiten Anzeigeelementen vorgesehen sind. Die ersten Anzeigeelemente 30 in schwarzer Farbe sind in einer ersten Zone (mittlerer Kreisring) angeordnet und die ersten Anzeigeelemente 40 in roter Farbe in einer zweiten Zone (innerer Kreisring). Die zweite Zone dient dazu, eine Überschreitung einer vorgegebenen dritten Zeitspanne darzustellen, wobei die dritte Zeitspanne durch die Gesamtheit aller ersten Anzeigeelemente 30 auf dem gesamten mittleren Kreisring dargestellt wird. Nach Überschreitung des Zeitpunktes an, dem alle zweiten Anzeigeelemente 30 auf dem mittleren Kreis vollständig dargestellt sind, werden die zweiten Anzeigeelemente 40 auf dem inneren Kreis gemäß Zeitfortschritt sukzessive dargestellt.

Ferner befindet sich in der Mitte der Anzeige 1 eine dreistellige Digitalanzeige 50, die je nach Modus unterschiedliche Bedeutungen hat. Links neben der Digitalanzeige ist ein Symbol 52 für eine Kartusche gezeigt. Bei gleichzeitiger Anzeige dieses Symbols und der Digitalanzeige wird mit letzterer die Anzahl der sich noch in der Kartusche befindlichen Tabletten angezeigt. Dieses Symbol kann sowohl in der Daueranzeigestellung als auch blinkend angezeigt werden, letzteres dann, wenn sich keine Tabletten mehr in der Kartusche befinden. Zusätzlich ist rechts neben der Digitalanzeige ein Symbol 54 für einen Tageskalender gezeigt. Bei gleichzeitiger Anzeige dieses Symbols und der Digitalanzeige wird mit letzterer die Anzahl der zurückgelegten Tage im aktuellen Einnahmezyklus angezeigt.

Ferner befindet sich unter der Digitalanzeige 50 ein Symbol 60 für eine Einnahmeunterbrechung. Wenn dieses Symbol zusammen mit der Digitalanzeige erscheint, zeigt die Digitalanzeige die bereits zurückgelegten Tage in der Unterbrechungsphase an.

Ferner befindet sich links neben dem Symbol 60 für die Einnahmeunterbrechung ein Warn- und Hinweissymbol 65, das in unterschiedlichen Situationen angezeigt wird und zwar in einer Daueranzeigestellung oder in Blinkstellung. Dieses Symbol kann in der hier gezeigten Darstellung mit einer Durchstreichung oder auch ohne Durchstreichung gezeigt werden. In durchgestrichener Darstellung wird dieses Symbol zusammen mit dem Symbol für die Unterbrechungsphase angezeigt und weist dann darauf hin, dass eine Unterbrechungsphase (noch) nicht eingeleitet werden darf.

Ferner befindet sich rechts neben dem Symbol 60 für die Einnahmeunterbrechung ein Bestätigungssymbol 70, das beispielsweise dann erscheint, wenn eine Entnahme einer Tablette als Einnahme bestätigt wird.

Oberhalb der Digitalanzeige 50 befindet sich ein Hinweissymbol 80 zur Anzeige, dass eine akustische Warnanzeige aktiviert (ohne Durchstreichung) oder deaktiviert (mit Durchstreichung, wie in der Fig. dargestellt) ist. Rechts oben sind ein Alarmsymbol 82 als Aufforderung an die Nutzerin, die Bedienungsanleitung zu Rate zu ziehen, und links ein Wamsymbol 84 über einen niedrigen Batterieladezustand gezeigt. Diese beiden Symbole sind nicht in schwarzer Farbe sondern vorzugsweise in roter Farbe oder in einer anderen Signalfarbe dargestellt, um den Warncharakter dieser Anzeigen zu unterstreichen. Außerdem kann das Alarmsymbol 82 nicht nur in einer Daueranzeige sondern auch als blinkende Anzeige wiedergegeben werden, letzteres dann, wenn die Nutzerin die Unterbrechungsphase während der obligatorischen Einnahmephase zu initiieren versucht.

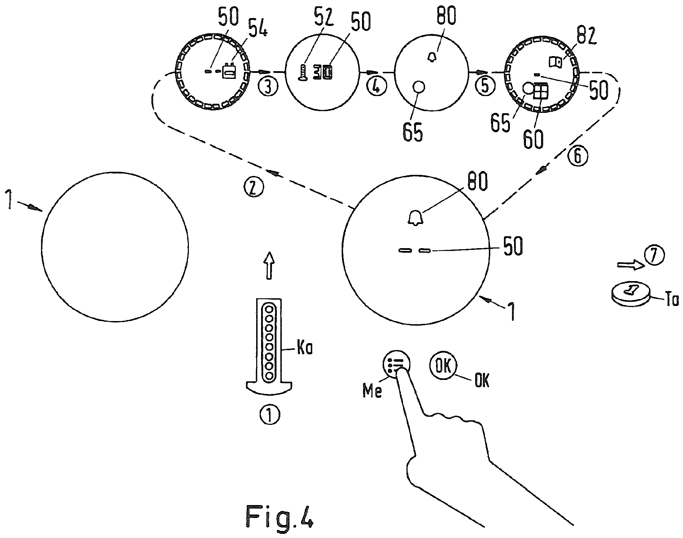

Fig. 4 zeigt die Anzeige 1 der erfindungsgemäßen Anzeigeeinrichtung zunächst vor dem ersten Einsetzen einer mit Tabletten gefüllten Kartusche in den Spender und dann nach dem Einsetzen.

Bevor in einen Spender zum ersten Mal eine Kartusche eingesetzt wird, ist auf der Anzeige 1 kein Anzeigeelement und auch kein Symbol zu sehen (linke Darstellung), weil der Spender in diesem Falle so ausgelegt ist, dass die Batterie im Spender mit der Anzeigeeinrichtung noch nicht verbunden ist. Anschließend wird die Kartusche Ka in den Spender eingeschoben (Schritt 1). Dadurch werden der Spender und die Anzeigeeinrichtung aktiviert. Es erscheint die Startdarstellung auf der Anzeige 1 (Default Screen). Auf der Anzeige werden zunächst lediglich die akustische Hinweisanzeige 80 und stellvertretend für eine digitale Zahlendarstellung durch die Digitalanzeige 50 standardmäßig zwei waagerechte Striche wiedergegeben. Diese Darstellung ist die Standarddarstellung der Anzeige.

Indem nun ein- oder mehrmals die Menütaste Me bestätigt wird, werden die verfügbaren Modi auf der Anzeige 1 nacheinander angezeigt:
Nach einmaligen Drücken der Menütaste Me (Schritt 2) erscheint in einer ersten Ebene der erste Anzeigemodus, in dem die in einem Einnahmezyklus bereits verstrichenen Tage angezeigt werden: Neben den in diesem Stadium noch standardmäßig angezeigten zwei Strichen der Digitalanzeige 50 ist das Kalendersymbol 54 angezeigt. Damit soll in diesem Stadium vor der ersten Entnahme einer Tablette aus dem Spender symbolisiert werden, dass noch kein Wert für die Anzahl der zurückgelegten Tage in einem Einnahmezyklus angezeigt werden kann.

Nach zweimaligem Drücken der Menütaste Me (Schritt 3) erscheint in einer zweiten Ebene der zweite Anzeigemodus, in dem die Anzahl der sich in der Kartusche noch befindenden Tabletten angezeigt werden: Neben der Digitalanzeige 50 erscheint das Kartuschensymbol 52. Dieses soll angeben, dass sich im vorliegenden Falle 30 Tabletten in der eingeschobenen Kartusche befinden. Das Kartuschensymbol wird in einer Daueranzeigestellung - nicht blinkend - wiedergegeben. Erst wenn die Anzahl der Tabletten beispielsweise 6 unterschreitet (voreingestellter Wert), blinkt das Kartuschensymbol. Außerdem wird in diesem Falle im Default Screen das Kartuschensymbol eingeblendet, das anzeigen soll, dass nur noch wenige Tabletten in der Kartusche vorhanden sind.

Nach dreimaligem Drücken der Menütaste Me (Schritt 4) erscheint in einer dritten Ebene der dritte Anzeigemodus, in dem der akustische Alarmmodus ein- oder ausgeschaltet werden kann: Zusätzlich zum Hinweis der akustischen Hinweisanzeige 80 ist das Wam-/Hinweissymbol 65, das blinkend dargestellt ist, gezeigt.

Nach viermaligem Drücken der Menütaste Me (Schritt 5) erscheint in einer vierten Ebene der vierte Anzeigemodus, in dem die Unterbrechungsphase eingeleitet bzw. angezeigt wird: Unten in der Mitte der Anzeige 1 ist das Symbol 60 für die Einnahmeunterbrechung angezeigt. In der Mitte befindet sich die Digitalanzeige 50, die im vorliegenden Fall anstelle einer einstelligen Zahl für die Anzahl der Unterbrechungstage einen einzigen waagerechten Strich zeigt. Rechts oben sind das Alarmsymbol 82 und links unten das Wam-/Hinweissymbol 65 mit durchgestrichener OK-Anzeige dargestellt, denn in der vorliegenden Phase steht die Nutzerin noch am Beginn der Einnahmephase und kann somit noch nicht die Unterbrechungsphase einleiten.

Die letztgenannten beiden Ebenen (dritte und vierte Ebene) können auch miteinander vertauscht sein.

Nach erneutem Betätigen der Menütaste Me (Schritt 6) kehrt die Anzeigeeinrichtung wieder in den Modus der Standarddarstellung zurück. Gleichfalls würde die Anzeigeeinrichtung von einem der ersten bis vierten Anzeigemodi dann wieder von selbst in den Modus der Standardeinstellung zurückkehren, wenn die Menütaste während eines Zeitraumes von 3 Sekunden nicht betätigt wird.

Die Nutzerin kann nun eine Tablette Ta aus dem Spender entnehmen (Schritt 7). Durch die Entnahme der Tablette wird das Einnahmeregime gestartet. Insbesondere wird zunächst das zweite Visualisierungsmittel gestartet:

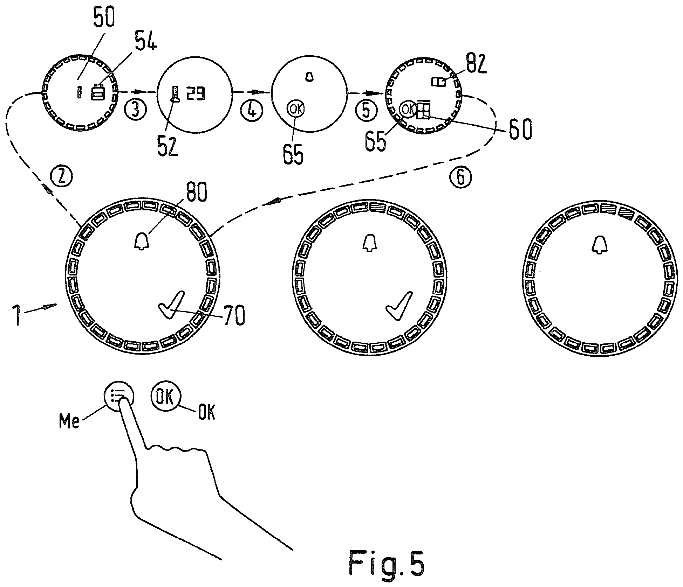

In Fig. 5 ist die Anzeige 1 der Anzeigeeinrichtung gezeigt, nachdem die erste Tablette entnommen worden ist. Falls die Tablette beispielsweise um 7:00 entnommen worden ist, stellt diese Zeit den zweiten Referenz-Einnahmezeitpunkt dar. Auf der Anzeige erscheint ein Kreis von 24 Anzeigerahmen 210 des zweiten Visualisierungsmittels, die jeweils 1 Stunde symbolisieren. Wie in der linken unteren Darstellung erkennbar ist, ist unmittelbar nach der Entnahme der ersten Tablette noch keiner der Anzeigerahmen 210 mit einer Anzeigefläche gefüllt. Ferner zeigt die Anzeigeeinrichtung das Bestätigungssymbol 70 sowie das Hinweissymbol 80 für die akustische Warnanzeige. Das Bestätigungssymbol gibt an, dass eine Tablette für eine Einnahme erfolgreich entnommen worden ist.

Nach wiederholtem Betätigen der Menütaste Me werden folgende Anzeigen in den ersten bis vierten Anzeigemodi dargestellt:
Nach einmaligem Betätigen (Schritt 2) erscheint der erste Anzeigemodus, in dem angezeigt wird, dass im aktuellen Einnahmezyklus 1 Tag verstrichen ist. Nach zweimaligem Betätigen (Schritt 3) erscheint der zweite Anzeigemodus, in dem angezeigt wird, dass sich in der Kartusche noch 29 Tabletten befinden. Nach dreimaligem Betätigen (Schritt 4) erscheint der dritte Anzeigemodus, in dem der akustische Alarmmodus ein- oder ausgeschaltet werden kann. Nach viermaligem Betätigen (Schritt 5) erscheint der vierte Anzeigemodus, in dem die Unterbrechungsphase eingeleitet bzw. angezeigt wird. Allerdings ist dies in der momentanen Phase nicht möglich, da sich die Nutzerin nach der Einnahme von nur 1 Tablette noch in der obligatorischen Einnahmephase befindet (Warn-/Hinweissymbol 65 durchgestrichen).

Nach erneutem Betätigen der Menütaste Me (Schritt 6) kehrt die Anzeige 1 wieder in den Modus der Standarddarstellung zurück. Bei fortschreitendem Zeitablauf nach der Entnahme und Einnahme der ersten Tablette erscheinen die beiden folgenden Darstellungen: In der mittleren unteren Darstellung ist angezeigt, dass seit der Entnahme etwa 1 Stunde vergangen ist (erster mit einer Anzeigefläche ausgefüllter Anzeigerahmen 210 des zweiten Visualisierungsmittels). In der rechten unteren Darstellung ist angezeigt, dass seit der Entnahme etwa 2 Stunden vergangen sind (zwei mit Anzeigeflächen ausgefüllte Anzeigerahmen 210).

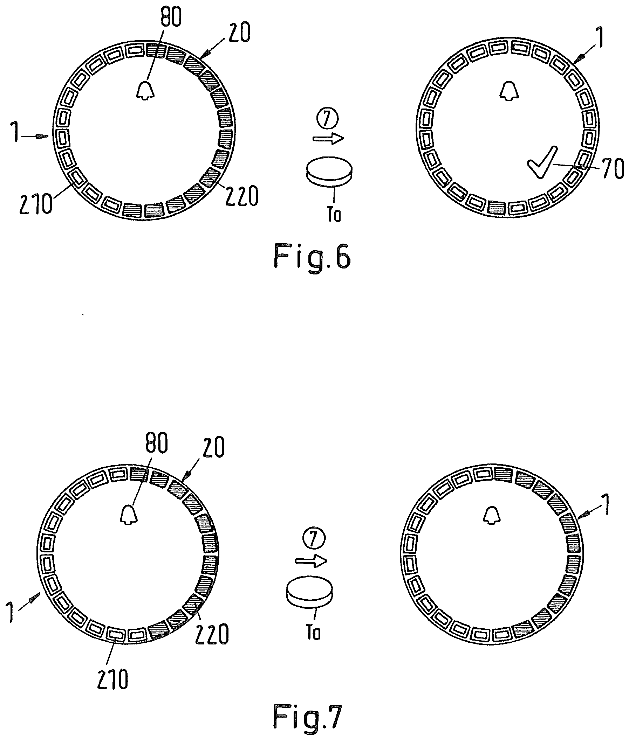

Der weitere Fortschritt des Zeitablaufs ist beispielhaft in Fig. 6 in der Standarddarstellung gezeigt: In der linken Darstellung der Anzeige 1 sind seit dem letzten zweiten Referenz-Einnahmezeitpunkt ca. 13 Stunden vergangen.

Die Tablette muss nicht zwingend zum Referenz-Einnahmezeitpunkt eingenommen werden. Es reicht aus, wenn sie innerhalb eines Einnahmezeitintervalls genommen wird. Im vorliegenden Fall kann sie beispielsweise innerhalb eines Zeitintervalls von 12 Stunden vor bis 2 Stunden nach dem Referenz-Einnahmezeitpunkt genommen werden. Die Tablette Ta wird im vorliegenden Fall innerhalb des Einnahmezeitintervalls von 12 Stunden vor dem Referenz-Einnahmezeitpunkt genommen (Schritt 7). Daher erscheint das Bestätigungssymbol 70 in der daraufhin erscheinenden Anzeige 1. Gleichzeitig wird der verbleibende Zeitraum bis zum nächsten Referenz-Einnahmezeitpunkt im Sinne einer "negativen Zeit" dargestellt, indem zunächst die Anzeigerahmen 210 mit Anzeigeflächen ausgefüllt werden, die auf dem sich vom Zeitpunkt der Entnahme und Einnahme (- 11 Stunden) bis zum aktuellen zweiten Referenz-Einnahmezeitpunkt erstreckenden Kreis liegen. Anschließend werden die Anzeigerahmen wieder auf normale Art und Weise, ausgehend vom ersten Anzeigeelement oben, mit Anzeigeflächen ausgefüllt.

Anders verhält es sich, wenn die Tablette Ta bereits vorzeitig aus dem Spender entnommen wird, d.h. vor Beginn des Einnahmezeitintervalls. Dies ist in Fig. 7 exemplarisch gezeigt. In diesem Falle beträgt die zweite Zeitspanne seit dem letzten zweiten Referenz-Einnahmezeitpunkt nur 11 Stunden. Somit wird eine nachfolgende Entnahme einer Tablette Ta (Schritt 7) nicht als wirksame Einnahme gebucht (keine Anzeige des Bestätigungssymbols, keine Änderung der Anzeige im zweiten Visualisierungsmittel).

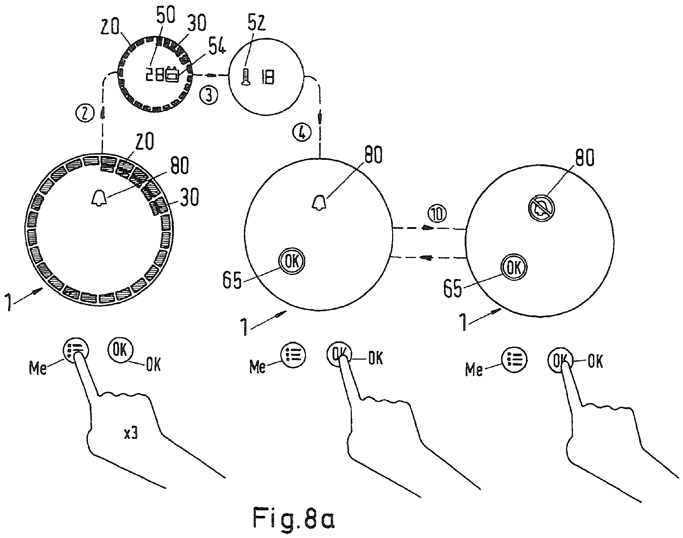

In Fig. 8a ist die Anzeige 1 der Anzeigeeinrichtung nach dem Überschreiten des ersten Referenz-Einnahmezeitpunktes gezeigt (Darstellung links unten). In diesem Falle sind nicht nur die vollständig angezeigten zweiten Anzeigeelemente 20 des zweiten Visualisierungsmittels auf dem äußeren Kreis gezeigt, sondern auch 5 erste Anzeigeelemente 30, die symbolisieren, dass die Nutzerin auch nach Ablauf einer den ersten Referenz-Einnahmezeitpunkt um 5 Stunden überschreitenden ersten Zeitspanne noch keine Tablette entnommen hat. Durch mehrmaliges Betätigen der Menütaste Me gelangt die Nutzerin nacheinander in den ersten Anzeigemodus, in dem angezeigt ist, dass sich die Nutzerin am 28. Tag des Einnahmezyklus befindet (Schritt 2), in den zweiten Anzeigemodus, in dem angezeigt ist, dass sich noch 18 Tabletten in der Kartusche befinden (Schritt 3) sowie in den dritten Anzeigemodus, in dem die Möglichkeit besteht, die akustische Hinweisanzeige 80 ein- oder auszuschalten (Schritt 4). Im vorliegenden Fall ist die Hinweisanzeige eingeschaltet, denn das Hinweissymbol 80 ist nicht durchgestrichen. Durch Betätigen der Bestätigungstaste Ok wird der Zustand "akustische Warnanzeige aus" gewählt (Schritt 10). Durch erneutes Drücken kann diese Wahl wieder rückgängig gemacht werden. Zwischen diesen beiden Zuständen kann beliebig oft hin- und hergeschaltet werden.

Außerdem ist im vorliegenden Fall das Warn-/Hinweissymbol 65 im blinkenden Zustand gezeigt. Dies bedeutet, dass die Nutzerin in dieser Einnahmephase (flexible Einnahmephase) in die Unterbrechungsphase überwechseln kann.

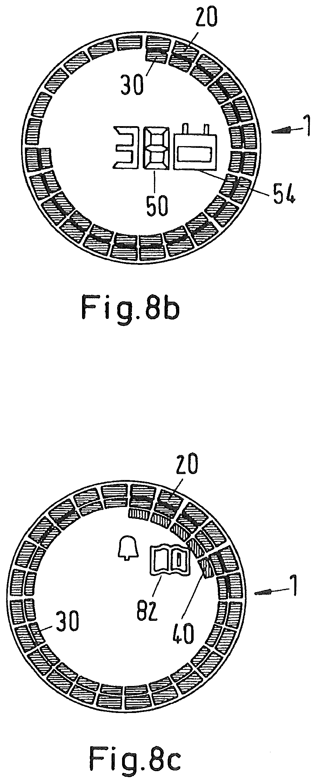

Weitere Darstellungen für eine Überschreitung des ersten Referenz-Einnahmezeitpunktes sind in Fig. 8b und 8c gezeigt: In Fig. 8b ist eine Überschreitung um 18 Stunden (zweite Anzeigeelemente 20 vollständig und 18 erste Anzeigeelemente 30 vorhanden) und in Fig. 8c eine Überschreitung um 29 Stunden (zweite Anzeigeelemente 20 vollständig, erste Anzeigeelemente 30 vollständig und 5 erste Anzeigeelemente 40 vorhanden) gezeigt. Die Anzeige in Fig. 8b gibt außerdem an, dass sich die Nutzerin am 38. Tag des Einnahmezyklus', d.h. in der flexiblen Einnahmephase, befindet. Im Falle der Fig. 8c wird die Nutzerin wegen der erheblichen Überschreitung des ersten Referenz-Einnahmezeitpunktes um 29 Stunden außerdem darauf hingewiesen, die Bedienungsanleitung zu Rate zu ziehen (Alarmsymbol 82).

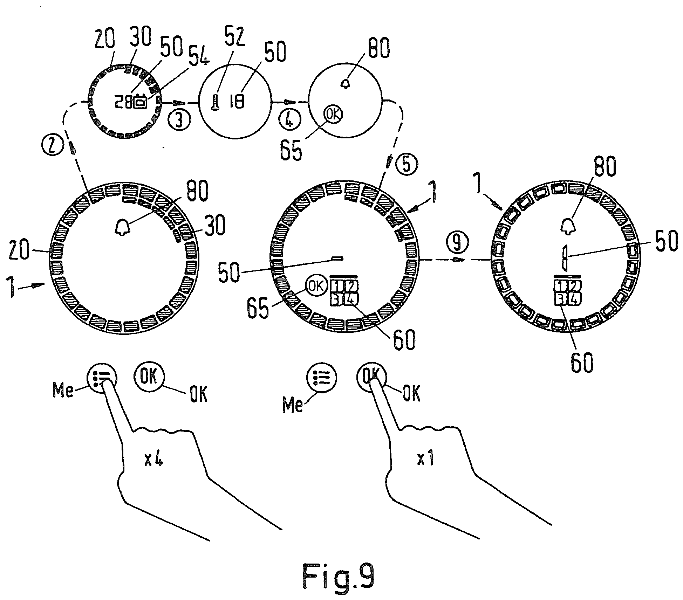

In Fig. 9 ist der Übergang in die Unterbrechungsphase gezeigt:

In der linken unteren Darstellung der Anzeige 1 ist gezeigt, dass die Nutzerin den ersten Referenz-Einnahmezeitpunkt um 5 Stunden überschritten hat. Dies ist daran zu erkennen, dass alle ersten Anzeigeelemente 20 des ersten Visualisierungsmittels sowie 5 zweite Anzeigeelemente 30 des zweiten Visualisierungsmittels angezeigt sind. Durch mehrmaliges Betätigen der Menütaste Me wird die Anzeigeeinrichtung durch die ersten bis dritten Anzeigemodi (Schritte 2, 3, 4, 5) zum vierten Anzeigemodus geschaltet. In diesem Anzeigemodus sind das Symbol für die Einnahmeunterbrechung 60, das Wam-/Hinweissymbol 65 sowie die Digitalanzeige 50 dargestellt. Das Warn-/Hinweissymbol zeigt an, dass eine Einleitung der Unterbrechungsphase möglich ist. Dies ist der Fall, weil sich die Nutzerin am 28. Tag des Einnahmezyklus' befindet (siehe Darstellung des ersten Anzeigemodus'). Durch Betätigen der Bestätigungstaste Ok wird die Unterbrechungsphase eingeleitet (Schritt 9). Daraufhin wird mit der Digitalanzeige 50 auf der Anzeige 1 angegeben, dass sich die Nutzerin am ersten Tag der Unterbrechungsphase befindet.

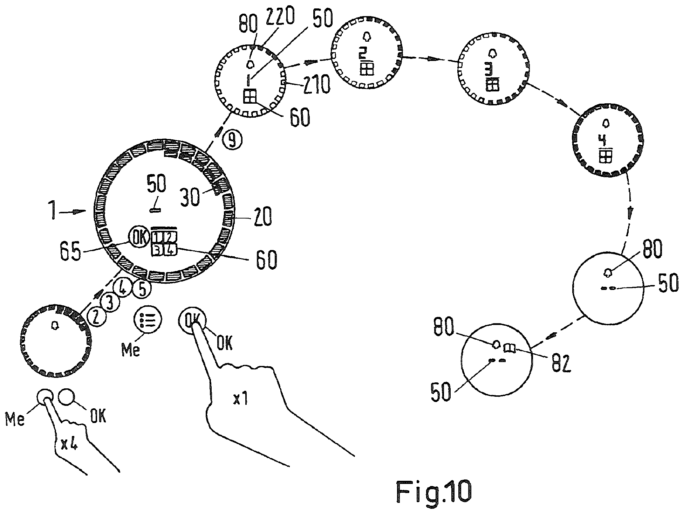

In der Unterbrechungsphase sind auf der Anzeige 1 nacheinander folgende Darstellungen erkennbar (Fig. 10):
Ausgehend von der Einnahmephase, in der die links unten klein dargestellte Anzeige 1 in der Standarddarstellung gezeigt ist, wird der vierte Anzeigemodus durch Betätigen der Menütaste Me gewählt (Schritte 2, 3, 4, 5). Es erscheint die Darstellung, in der die Nutzerin die Wahl hat, die Unterbrechungsphase einzuleiten. Dies ist durch das Symbol 60 für die Einnahmeunterbrechung angezeigt. Außerdem ist mit dem Wam-/Hinweissymbol 65 bestätigt, dass der Start der Unterbrechungsphase möglich ist. Außerdem geben die Anzeigeelemente 20 des zweiten Visualisierungsmittels an, dass 5 Stunden an diesem ersten Tag verstrichen sind. Es handelt sich um den seit dem ersten Referenz-Einnahmezeitpunkt verstrichenen Zeitraum (siehe Zeitanzeige des zweiten Visualisierungsmittels auf der Anzeige gemäß großer Darstellung der Anzeige). Durch Betätigen der Bestätigungstaste Ok (Schritt 9) wird in die Unterbrechungsphase gewechselt. Die Digitalanzeige 50 gibt nun an, dass sich die Nutzerin am ersten Tag der Unterbrechungsphase befindet.

Während der Unterbrechungsphase nimmt die Nutzerin keine Tabletten. Die Anzeige 1 zeigt mit der Digitalanzeige 50 sowie mit den zweiten Anzeigeelementen 20 des zweiten Visualisierungsmittels den jeweils zurückgelegten Zeitraum an. In den hier gezeigten Darstellungen sind seit dem Referenz-Einnahmezeitpunkt am ersten Tag 5 Stunden (d.h. insgesamt 5 Stunden), am zweiten Tag 9 Stunden (d.h. insgesamt 33 Stunden), am dritten Tag 13 Stunden (d.h. insgesamt 61 Stunden) und am vierten Tag 23 Stunden (d.h. insgesamt 95 Stunden) vergangen. Unmittelbar danach schaltet die Anzeige auf die Startdarstellung um. Wird in dieser Situation eine neue Tablette entnommen, so ergibt sich der Ablauf, der auch bereits an Hand von Fig. 4, 5 beschrieben worden ist. Wird dagegen keine Tablette entnommen, so erscheint das Alarmsymbol 82 in Daueranzeigestellung (letzte Darstellung in der Reihe der Darstellungen in Fig. 10). Damit wird die Nutzerin aufgefordert, die Bedienungsanleitung zu Rate zu ziehen. Außerdem kann ein Signalton ertönen, um die Nutzerin an den Start der neuen Einnahmephase zu erinnern, beispielsweise während weniger Sekunden, wobei der Signalton mehrfach in einem bestimmten Rhythmus wiederholt wird, etwa im Abstand von 15 Minuten.

Bei der Entnahme von mehreren Tabletten kurz hintereinander, wird die zweite Entnahme nicht als Einnahme berücksichtigt (Fig. 11):
Wird beispielsweise bei Überschreitung des ersten Referenz-Einnahmezeitpunktes um 7 Stunden (Fig. 11a: linke Darstellung: zweite Anzeigeelemente 20 (äußerer Ring) vollständig und 7 erste Anzeigeelemente 30 (innerer Ring) vorhanden) eine erste Tablette Ta genommen (Schritt 7), so schaltet die Einrichtung auf eine Anzeige um, der zufolge seit dem letzten zweiten Referenz-Einnahmezeitpunkt 7 Stunden vergangen sind (Fig. 11a: mittlere Darstellung: 7 zweite Anzeigeelemente 20 vollständig vorhanden). Die Entnahme wird in diesem Falle mit dem Bestätigungssymbol 70 bestätigt. Bei erneuter Entnahme einer Tablette Ta wird diese jedoch nicht mehr als Einnahme berücksichtigt (Fig. 11 a: rechte Darstellung: keine weitere Veränderung der Anzeigeelemente 20, keine Anzeige des Bestätigungssymbols).

In einer anderen Konstellation sind seit dem ersten Referenz-Einnahmezeitpunkt 30 Stunden verstrichen (Fig. 11 b: linke Darstellung: zweite Anzeigeelemente 20 (äußerer Ring) vollständig, erste Anzeigeelemente 30 (mittlerer Ring) vollständig und 6 erste Anzeigeelemente 40 (innerer Ring) vorhanden). In diesem Falle wird das Alarmsymbol 82 wegen der erheblichen Überschreitung des ersten Referenz-Einnahmezeitpunktes eingeblendet. Bei einer Entnahme einer Tablette Ta (Schritt 7) schaltet die Einrichtung auf eine Anzeige um, der zufolge seit dem letzten zweiten Referenz-Einnahmezeitpunkt 6 Stunden vergangen sind (Fig. 11 b: mittlere Darstellung: 6 zweite Anzeigeelemente 20 vollständig vorhanden). Die Entnahme wird mit dem Bestätigungssymbol 70 bestätigt. Allerdings wird die Nutzerin in dieser Situation aufgefordert, die Bedienungsanleitung zu Rate zu ziehen (Alarmsymbol 82 angezeigt). Bei erneuter Entnahme einer Tablette Ta wird diese jedoch nicht mehr als Einnahme berücksichtigt (Fig. 11b: rechte Darstellung: keine weitere Veränderung der Anzeigeelemente 20, kein Bestätigungssymbol).

Bei der Entnahme von mehreren Tabletten, die unterschiedlichen Referenz-Einnahmezeitpunkten zugeordnet werden können, wird eine zweite Entnahme einer Tablette dagegen als Einnahme berücksichtigt (Fig. 12):
Wird beispielsweise bei Überschreitung des ersten Referenz-Einnahmezeitpunktes um 15 Stunden (Fig. 12a: linke Darstellung: zweite Anzeigeelemente 20 vollständig und 15 erste Anzeigeelemente 30 vorhanden) eine erste Tablette Ta genommen (Schritt 7), so schaltet die Einrichtung auf eine Anzeige um, der zufolge seit dem letzten zweiten Referenz-Einnahmezeitpunkt 15 Stunden vergangen sind (Fig. 12a: mittlere Darstellung: 15 zweite Anzeigeelemente 20 vollständig vorhanden). Die Entnahme wird mit dem Bestätigungssymbol 70 bestätigt. Bei erneuter Entnahme einer Tablette Ta wird diese als weitere Einnahme berücksichtigt, weil diese zweite Entnahme innerhalb des Zeitintervalls des nächsten Referenz-Einnahmezeitpunktes liegt (Zeitintervall beginnt bei Referenz-Einnahmezeitpunkt minus 9 Stunden). Diese Einnahme wird wiederum mit dem Bestätigungssymbol 70 bestätigt. Außerdem zeigt die Anzeige 1 nun zunächst eine neue negative zweite Zeitspanne an: Fig. 12a, rechte Darstellung zeigt, dass die zweiten Anzeigeelemente 20, ausgehend von einem Anzeigeelement 20 "Referenz-Einnahmezeitpunkt minus 9 Stunden", zu zählen beginnen.

In einer anderen Konstellation sind seit dem ersten Referenz-Einnahmezeitpunkt 42 Stunden verstrichen (Fig. 12b: linke Darstellung: zweite Anzeigeelemente 20 vollständig, erste Anzeigeelemente 30 vollständig und 18 erste Anzeigeelemente 40 vorhanden). In diesem Falle wird das Alarmsymbol 82 wegen der erheblichen Überschreitung des ersten Referenz-Einnahmezeitpunktes eingeblendet. Bei einer Entnahme einer Tablette Ta (Schritt 7) schaltet die Einrichtung auf eine Anzeige um, der zufolge seit dem letzten zweiten Referenz-Einnahmezeitpunkt 18 Stunden vergangen sind (Fig. 12b: mittlere Darstellung: 18 zweite Anzeigeelemente 20 vollständig vorhanden). Die Entnahme wird mit dem Bestätigungssymbol 70 bestätigt. Allerdings wird die Nutzerin auch hier dazu aufgefordert, wegen der erheblichen Überschreitung des ersten Referenz-Einnahmezeitpunktes die Bedienungsanleitung zu Rate zu ziehen (Alarmsymbol 82 angezeigt). Bei erneuter Entnahme einer Tablette Ta wird diese als weitere Einnahme berücksichtigt, weil diese zweite Entnahme innerhalb des Zeitintervalls des nächsten Referenz-Einnahmezeitpunktes liegt (Zeitintervall beginnt bei Referenz-Einnahmezeitpunkt minus 12 Stunden). Diese Einnahme wird wiederum mit dem Bestätigungssymbol 70 bestätigt. Außerdem zeigt die Einrichtung nun zunächst eine neue negative zweite Zeitspanne an: Fig. 12b, rechte Darstellung zeigt, dass die zweiten Anzeigeelemente, ausgehend von einem Anzeigeelement 20 "Referenz-Einnahmezeitpunkt minus 6 Stunden", zu zählen beginnen.

Versäumt es die Nutzerin, die Tabletten während einer längeren Zeitspanne zu nehmen, so startet die Anzeigeeinrichtung automatisch die Unterbrechungsphase. In Fig. 13 ist gezeigt, auf welche Art und Weise die Einrichtung von der flexiblen Phase automatisch in die Unterbrechungsphase wechselt:
Ausgehend von einer Darstellung, gemäß der der erste Referenz-Einnahmezeitpunkt bereits 47 Stunden zurückliegt (linke große Darstellung: zweite Anzeigeelemente 20 vollständig, erste Anzeigeelemente 30 vollständig und 23 erste Anzeigeelemente 40 vorhanden) und die Nutzerin mit dem Alarmsymbol 82 darauf hingewiesen wird, die Bedienungsanleitung zu Rate zu ziehen, wechselt die Einrichtung 2 Stunden später zu der rechts daneben (kleiner) dargestellten Anzeige 1 automatisch über, wonach sich die Nutzerin nunmehr in der Unterbrechungsphase befindet. Dies ist an dem Symbol 60 für die Einnahmeunterbrechung sowie daran zu erkennen, dass die Digitalanzeige 50 nur einstellig erscheint. Die Digitalanzeige gibt an, dass sich die Nutzerin bereits am dritten Tage der Unterbrechung befindet. Dies ergibt sich aus dem längeren Zeitverzug bei der Entnahme von Tabletten von mittlerweile über 48 Stunden. Die weiteren Darstellungen in Fig. 13 zeigen die Situationen jeweils am vierten Unterbrechungstag (5 Stunden nach dem Referenz-Einnahmezeitpunkt), unmittelbar nach Beendigung des Unterbrechungszeitraumes und nach weiterem Zeitablauf, ohne dass die Nutzerin eine Tablette entnommen hat.

Fig. 14 zeigt einen derartigen Übergang in die Unterbrechungsphase auch für den Fall, dass es die Nutzerin versäumt hat, die Tabletten in der obligatorischen Einnahmephase zu nehmen. Da die Kontrazeption in dieser Situation wegen der versäumten Tabletteneinnahmen ohnehin nicht sicher ist, wechselt die Anzeigeeinrichtung in die Unterbrechungsphase, um die Nutzerin wieder in die Einnahmephase zu leiten. Die Anzeige unterscheidet sich in diesem Falle nicht von derjenigen, die beim Wechsel von der flexiblen Phase in die Unterbrechungsphase erscheint.

In Fig. 15 ist die Anzeige 1 bei niedrigem Batterie-Ladezustand wiedergegeben. In der linken Darstellung von Fig. 15 ist das Warnsymbol 84 für einen niedrigen Batterieladezustand gezeigt. Die Anzeige gibt an, dass sich die Nutzerin in einer Einnahmephase befindet (kein Symbol für die Unterbrechungsphase). Der Ladezustand der Batterie ist stets so bemessen, dass der Einnahmezyklus noch bis zu Ende geführt werden kann. Daher wird die Nutzerin die Einnahmephase noch bis zum Ende der Unterbrechungsphase führen können (mittlere Darstellung). Nach Beendigung der Unterbrechungsphase wechselt die Anzeigeeinrichtung jedoch nicht zur Standarddarstellung sondern schaltet die Anzeige ab (rechte Darstellung).

In Fig. 16 ist eine Anzeige einer erfindungsgemäßen Anzeigeeinrichtung in einer zweiten Ausführungsform gezeigt. Anstelle der ersten sektoriellen Anzeigeelemente 30, 40 und der zweiten sektoriellen Anzeigeelemente 20 auf drei Kreisen sind hier lediglich sektorielle erste Anzeigeelemente, die auf zwei Kreisen liegen, erkennbar, weil beide Typen von Anzeigeelementen in dieser Ausführungsform nicht gleichzeitig erscheinen. Während die zweiten Anzeigeelemente des ersten Typs 30 schwarz gehalten sind, sind die zweiten Anzeigeelemente des zweiten Typs 40 rot gefärbt, um der Nutzerin eine erhebliche Zeitüberschreitung zu signalisieren.

Außerdem unterscheiden sich in diesem Falle die Gestaltungen des Symbols 60 für die Einnahmeunterbrechung, des Warn- und Hinweissymbols 65, des Symbols 54 für den Tageskalender und des Hinweissymbols 80 für die Aktivierung bzw. Deaktivierung der Warnanzeige von den entsprechenden Symbolen der ersten Ausführungsform (siehe Fig. 3). Ein Bestätigungssymbol ist nicht vorgesehen. Zudem unterscheidet sich die Anordnung der Symbole von der der ersten Ausführungsform geringfügig.

In Fig. 17 ist die Startdarstellung auf der Anzeige 1 (Default Screen) in verschiedenen Stadien des Zeitfortschrittes gezeigt: Die zweiten sektoriellen Anzeigeelemente 20, die den Zeitfortschritt seit dem zweiten Referenz-Einnahmezeitpunkt (symbolisiert durch das außen liegende große Dreieck 90) anzeigen, stehen jeweils für 1 Stunde. Diese Anzeigeelemente bestehen jeweils aus im wesentlichen rechteckigen Rahmen. Zunächst werden alle Anzeigeelemente eingeschaltet, um einer Nutzerin zu signalisieren, dass noch der gesamte Zeitraum bis zum nächsten Referenz-Einnahmezeitpunkt zur Verfügung steht. Im vorliegenden Fall ist gemäß der Darstellung in Fig. 17(a) bereits 1 Stunde seit dem zweiten Referenz-Einnahmezeitpunkt vergangen. Da die letzte Tablette kurz vor der Aufnahme dieser Darstellung genommen wurde, zeigt die Anzeige bereits nur noch 23 Anzeigeelemente an, um signalisieren, dass nur noch 23 Stunden bis zum nächsten Referenz-Einnahmezeitpunkt verbleiben. Bei Aufnahme der nächsten Darstellung (Fig. 17(b)) sind bereits 6 Stunden vergangen, so dass auch 6 Anzeigeelemente ausgeschaltet sind. Es verbleiben also noch 18 Stunden bis zum nächsten Referenz-Einnahmezeitpunkt. Fig. 17(c), (d) und (e) zeigen entsprechende Darstellungen 13 Stunden, 2 Stunden bzw. 1 Stunde vor dem nächsten Referenz-Einnahmezeitpunkt.

Falls die nächste Tablette noch vor dem Erreichen des zweiten Referenz-Einnahmezeitpunktes 90 genommen wird, muss eine verlängerte Restzeitspanne bis zum nächsten zweiten Referenz-Einnahmezeitpunkt für die Einnahme der darauf folgenden Tablette angezeigt werden. Dies kann beispielsweise dadurch realisiert werden, dass nach der Entnahme der Tablette alle zweiten Anzeigeelemente 20 des zweiten Visualisierungsmittels angezeigt werden und darüber hinaus weitere Anzeigeelemente, die beispielsweise auf einem gegenüber dem äußeren Kreis nach innen versetzten Kreis liegen und dem 24 Stunden überschießenden Restzeitraum entsprechen (nicht dargestellt). Wird die nächste Tablette beispielsweise 2 Stunden vor dem zweiten Referenz-Einnahmezeitpunkt genommen (entsprechend Fig. 17(d)), so würden nach der Entnahme der Tablette alle Anzeigeelemente 20 auf dem äußeren Kreis angezeigt und zusätzlich auf einem weiter innen liegenden Kreis weitere 2 Anzeigeelemente, die sich an der Stelle der in Fig. 17(d) gezeigten Anzeigeelemente, allerdings nach innen versetzt, befinden. Im weiteren zeitlichen Verlauf würden dann lediglich zunächst diese beiden Anzeigeelemente sukzessive gelöscht und erst danach, beginnend mit einem ersten rechts neben der Marke 90 für den zweiten Referenz-Einnahmezeitpunkt liegenden Anzeigeelement, nach und nach abgeschaltet werden.

Fig. 18 zeigt wiederum die Startdarstellung auf der Anzeige 1 (Default Screen) in verschiedenen Stadien des Zeitfortschrittes im Anschluss an Fig. 17, in diesem Falle jedoch nach Verstreichen des nächsten Referenz-Einnahmezeitpunktes 90, ohne dass eine Tablette genommen wurde. Somit ist der jüngste Referenz-Einnahmezeitpunkt ein erster Referenz-Einnahmezeitpunkt. Nach Erreichen dieses Referenz-Einnahmezeitpunktes, ohne dass eine Tablette genommen wurde, sind die zweiten Anzeigeelemente gelöscht. Nach weiterem Zeitablauf nach dem ersten Referenz-Einnahmezeitpunkt, erscheinen sukzessive erste Anzeigeelemente 30, die die Zeitüberschreitung angeben. In Fig. 18(a) ist dargestellt, dass seit dem ersten Referenz-Einnahmezeitpunkt 1 Stunde vergangen ist. In Fig. 18(b) - (e) sind weitere Zeitfortschritte dargestellt, nämlich 7 Stunden, 12 Stunden, 19 Stunden bzw. 23 Stunden seit dem ersten Referenz-Einnahmezeitpunkt. Zusätzlich gibt die Ziffernanzeige 50 an, dass 1 Tablette bisher nicht genommen worden ist. Weiterhin gibt das modifizierte Hinweissymbol 80 für die Aktivierung bzw. Deaktivierung der Warnanzeige (durch die links oben dargestellten Wellen) an, dass der Einnahmezeitpunkt überschritten ist.

Fig. 19 zeigt den weiteren Zeitfortschritt nach Ablauf von mehr als 24 Stunden seit dem ersten Referenz-Einnahmezeitpunkt an. In Fig. 19(a) ist die Darstellung gezeigt, die sich 24 Stunden + 1 Stunde (= 25 Stunden) seit dem ersten Referenz-Einnahmezeitpunkt ergibt. Hierzu ist nun zusätzlich zu den ersten Anzeigeelementen 30 auf dem äußeren Kreis ein weiteres erstes Anzeigeelement 40 auf einem weiter innen liegenden Kreis angezeigt. Dieses weitere Anzeigeelement ist vorzugsweise in roter Farbe gehalten, um die Nutzerin vor einer weiteren Zeitüberschreitung zu warnen. Weiterhin zeigt die Digitalanzeige 50 an, dass nunmehr bereits 2 Tabletten nicht genommen wurden. In den weiteren Darstellung gemäß Fig. 19(b) und 19 (c) sind seit dem ersten Referenz-Einnahmezeitpunkt 24 + 9 Stunden (= 33 Stunden) bzw. 24 + 20 Stunden (= 44 Stunden) vergangen.

In Fig. 20 ist die Anzeige 1 dargestellt, die sich vor (Fig. 20(a)) bzw. nach (Fig. 20(b)) der Entnahme einer einzelnen Tablette ergibt. Fig. 20(a) entspricht der Darstellung in Fig. 19(c). Vor der Entnahme einer Tablette hat die Anzeige signalisiert, dass 2 Tabletten nicht genommen worden sind. Nach der Entnahme von 1 Tablette zeigt nun die Anzeige an, dass noch 1 Tablette genommen werden muss. Dies ist zum einen an der Digitalanzeige 50 erkennbar sowie an der Löschung der ersten Anzeigeelemente 40 sowie der partiellen Löschung der ersten Anzeigeelemente 30. Nun müsste noch 1 weitere Tablette genommen werden, um den erforderlichen Rhythmus wieder einzuhalten. Wird diese weitere Tablette genommen, werden auch die ersten Anzeigeelemente 30 gelöscht, und es werden (in vorliegenden Fall) noch 4 zweite Anzeigeelemente gezeigt (nicht dargestellt), die den Zeitraum bis zum nächsten Referenz-Einnahmezeitpunkt anzeigen.

In Fig. 21 ist die Anzeige 1 wiedergegeben, die sich ergibt, nachdem die Unterbrechungsphase eingeleitet worden ist. In diesem Falle erscheinen das Symbol 60 für die Einnahmeunterbrechung sowie das Warn- und Hinweissymbol 65. Unmittelbar nach dem Einleiten der Unterbrechungsphase (Fig. 21 (a)) sind lediglich diese beiden Symbole angezeigt. Nach Anlauf von wenigen Stunden nach der Einleitung ist hier zusätzlich angezeigt, dass sich die Nutzerin am 1. Tag der Unterbrechung befindet und zwar nach Ablauf von 4 Stunden (Fig. 21(b)). In Fig. 21(c) ist die Anzeige wiedergegeben, die sich am 2. Tag der Unterbrechungsphase und zwar nach 24 + 9 Stunden (= 33 Stunden) nach der Einleitung ergibt. In Fig. 21 (d) ist die entsprechende Anzeige am 3. Tag der Unterbrechungsphase nach 24 + 24 + 9 Stunden (= 57 Stunden) nach der Einleitung gezeigt.

Für den Fall, dass die Tabletten für die Kontrazeption nicht regelmäßig genommen werden, kann die Verhütung unter bestimmten Bedingungen nicht mehr gewährleistet werden. Für diesen Fall ist es notwendig, dass die Nutzerin zusätzliche Kontrazeptiva anwendet. Dieses Erfordernis kann separat und konkret auf der Anzeige 1 mittels eines Warnsymbols wegen fehlender Kontrazeption angezeigt werden, beispielsweise durch ein Ausrufezeichen ("!"). Dieses Warnsymbol wird nur dann angezeigt, wenn keine ausreichende Verhütung mehr garantiert ist, und soll der Nutzerin signalisieren, dass nun zusätzliche Verhütungsmittel angewendet werden müssen. Ein derartiger Fall tritt dann ein, wenn die Tabletten unregelmäßig genommen wurden. Dabei kommt es darauf an, welche Zeiträume einerseits ordnungsgemäßer, d.h. regelmäßiger Einnahme, und andererseits unterbrochener Einnahme von Tabletten sich ergeben. In Fig. 22 sind Beispiele für Fehleinnahmen an bestimmten Tagen im Verlauf des Einnahmeregimes gezeigt (ordnungsgemäße Einnahme: "o", keine Einnahme. "x"), die in zwei Fällen zu einem Zustand mangelnder Kontrazeption führen. Daher werden in diesem Falle zusätzliche kontrazeptive Maßnahmen (Erfordernis zusätzlicher Anwendung von anderen Verhütungsmitteln) erforderlich. Hierzu wird das genannte Warnsymbol auf der Anzeige angezeigt:
In den Figuren ist jeweils ein Ausschnitt aus einem Einnahmekalender mit dem jeweils 17., 18., 19. Tag usw. im Einnahmeregime gezeigt (Tagesbenennung in der ersten Zeile). Eine Einnahme einer Tablette an dem jeweiligen Tag ist in der zweiten Zeile mit "o" dokumentiert. Falls keine Tablette genommen wurde, ist ein "x" angezeigt. In der dritten Zeile ist angegeben, ob an dem jeweiligen Tag zusätzliche kontrazeptive Maßnahmen erforderlich sind ("!") oder nicht (kein Eintrag in das zugehörige Feld). In ersteren Fällen erscheint dieses Warnsymbol auf der Anzeige.

In Fig. 22a ist ein Beispiel für eine Einnahme gezeigt, bei der die Einnahme einer Tablette am 28. und dann wieder am 36. Tag vergessen wurde. Zusätzliche kontrazeptive Maßnahmen sind in diesem Falle aus folgenden Gründen nicht erforderlich (kein Eintrag in der dritten Zeile): Lediglich an einem einzigen Tag innerhalb eines Zeitraumes von 7 Tagen mit unregelmäßiger Einnahme (beispielsweise im Zeitraum vom 28. Tag bis zum 34. Tag) wurde die Tablette nicht genommen, und vor diesem Tag liegt ein 7-tägiger Zeitraum (21. Tag bis 27. Tag), an dem die Tabletten regelmäßig genommen wurden. Ferner schließt sich an den Tag, an dem die Tabletten nicht genommen wurden, (28. Tag) ein weiterer 7-tägiger Zeitraum (29. Tag bis 35. Tag) an, in dem die Tabletten regelmäßig genommen wurden. Der Fehleinnahme-Zeitraum beträgt in diesem Falle lediglich 1 Tag (28. Tag). Der Umstand, dass später (am 36. Tag) wieder vergessen wurde, die Tablette zu nehmen, führt aus den vorgenannten Gründen nicht dazu, dass nun zusätzliche kontrazeptive Maßnahmen erforderlich wären. Der Zeitraum von 7 Tagen zwischen den beiden Tagen mit fehlender Einnahme (28. Tag und 36. Tag) garantiert die ovulationshemmende Wirkung. Aus diesem Grunde wird auf der Anzeige auch nicht das Warnsymbol ("!") aktiviert.

In Fig. 22b ist ein Beispiel für eine Einnahme gezeigt, bei der die Tabletten am 28. Tag und dann wieder am 34., 35., 36. und 37. Tag nicht genommen wurden. In diesem Falle sind zusätzliche kontrazeptive Maßnahmen erforderlich: Innerhalb eines Zeitraumes von 7 Tagen mit unregelmäßiger Einnahme, gerechnet von einem ersten Tag, an dem eine Tablette nach mindestens 7 Tage währender ununterbrochener Einnahme nicht genommen worden ist, (28. Tag), sind an 2 Tagen Tabletten nicht genommen worden, nämlich am 28. Tag und am 34. Tag. An den letzten Tag innerhalb dieses 7-tägigen Zeitraumes schließt sich nicht eine mindestens 7-tägige Zeitspanne an, in der ununterbrochen Tabletten genommen werden, denn auch am 35., am 36. und am 37. Tag werden Tabletten nicht genommen. Der Fehleinnahme-Zeitraum beträgt hier 10 Tage, denn er erstreckt sich vom 28. Tag bis zum 37. Tag. Der erste Tag, an dem zusätzliche kontrazeptive Maßnahmen erforderlich werden, ist der sich an den vorgenannten 7-tägigen Zeitraum mit unregelmäßiger Einnahme ab dem 28. Tag anschließende 8. Tag. Hierbei handelt es sich um den 35. Tag, denn der 7-tägige Zeitraum mit unregelmäßiger Einnahme endet am 34. Tag. Das Erfordernis zusätzlicher kontrazeptiver Maßnahmen dauert dann so lange an, bis wieder ein Zeitraum von mindestens 7 Tagen währender ununterbrochener Einnahme abgelaufen ist. Somit erstreckt sich der Zeitraum, in dem zusätzliche kontrazeptive Maßnahmen erforderlich sind, bis zum 44. Tag, denn eine 7-tägige ununterbrochene Einnahme ist erst am 44. Tag erreicht. Daher wird das Warnsymbol wegen fehlender Kontrazeption vom 35. Tag bis zum 44. Tag aktiviert.

In Fig. 22c ist ein Beispiel für eine Einnahme gezeigt, bei der die Tabletten am 34., 35., 36. und 37. Tag und dann wieder am 40. Tag nicht genommen wurden. In diesem Falle sind zusätzliche kontrazeptive Maßnahmen nicht erforderlich: Innerhalb eines Fehleinnahme-Zeitraumes von 7 Tagen, gerechnet von einem ersten Tag, an dem eine Tablette nach mindestens 7 Tage währender ununterbrochener Einnahme nicht genommen worden ist, (34. Tag), sind an 5 Tagen Tabletten nicht genommen worden, nämlich am 34. Tag, am 35. Tag, am 36. Tag, am 37. Tag und am 40. Tag. An den letzten Tag innerhalb dieses 7-tägigen Fehleinnahme-Zeitraumes, d.h. an den 40. Tag, schließt sich eine mindestens 7-tägige Zeitspanne an, in der ununterbrochen Tabletten genommen werden (ab dem 41. Tag). Daher wird das Warnsymbol nicht aktiviert.

In Fig. 22d ist ein Beispiel für eine Einnahme gezeigt, bei der die Tabletten am 34., 35., 36. und 37. Tag und dann wieder am 41. Tag nicht genommen wurden. In diesem Falle sind zusätzliche kontrazeptive Maßnahmen erforderlich: Innerhalb eines Zeitraumes von 7 Tagen mit unregelmäßiger Einnahme, gerechnet von einem ersten Tag, an dem eine Tablette nach mindestens 7 Tage währender ununterbrochener Einnahme nicht genommen worden ist, (34. Tag), sind an 4 Tagen Tabletten nicht genommen worden, nämlich am 34. Tag, am 35. Tag, am 36. Tag und am 37. Tag. An den letzten Tag innerhalb dieses 7-tägigen Zeitraumes (40. Tag) mit unregelmäßiger Einnahme schließt sich nicht eine mindestens 7-tägige Zeitspanne an, in der ununterbrochen Tabletten genommen werden, denn auch am 41. Tag wird eine Tablette nicht genommen. In der Tat dauert der Fehleinnahme-Zeitraum 8 Tage an. Der erste Tag, an dem zusätzliche kontrazeptive Maßnahmen erforderlich werden, ist der sich an den vorgenannten 7-tägigen Zeitraum mit unregelmäßiger Einnahme anschließende 8. Tag. Hierbei handelt es sich um den 41. Tag, denn der 7-tägige Zeitraum mit unregelmäßiger Einnahme endet am 40. Tag. Das Erfordernis zusätzlicher kontrazeptiver Maßnahmen dauert dann so lange an, bis wieder ein Zeitraum von mindestens 7 Tagen währender ununterbrochener Einnahme abgelaufen ist. Somit dauert der Zeitraum, in dem zusätzliche kontrazeptive Maßnahmen erforderlich sind, bis zum 48. Tag, denn eine 7-tägige ununterbrochene Einnahme ist erst am 48. Tag erreicht. Daher wird das Warnsymbol wegen fehlender Kontrazeption vom 41. Tag bis zum 48. Tag aktiviert.

Insbesondere umfasst die Erfindung folgende Aspekte:
1. Anzeigeeinrichtung für einen Spender (Sp) für Arzneimittelportionen (Ta), wobei die Arzneimittelportionen zu regelmäßig wiederkehrenden Referenz-Einnahmezeitpunkten einzunehmen sind und wobei die Anzeigeeinrichtung eine Anzeige (1) sowie eine elektronische Ansteuerung für die Anzeige umfasst, dadurch gekennzeichnet, dass die Anzeige ein erstes Visualisierungsmittel zur Darstellung einer ersten Zeitspanne zwischen einem ersten Referenz-Einnahmezeitpunkt und dem aktuellen Zeitpunkt umfasst und sich der erste Referenz-Einnahmezeitpunkt dadurch auszeichnet, dass bis zu dem ersten Referenz-Einnahmezeitpunkt keine Arzneimittelportion genommen worden ist.
2. Anzeigeeinrichtung für einen Spender (Sp) für Arzneimittelportionen (Ta) nach Aspekt 1, dadurch gekennzeichnet, dass die Anzeige (1) zusätzlich ein zweites Visualisierungsmittel zur Darstellung einer zweiten Zeitspanne zwischen einem zweiten Referenz-Einnahmezeitpunkt und dem aktuellen Zeitpunkt umfasst, wobei sich der zweite Referenz-Einnahmezeitpunkt dadurch auszeichnet, dass er in einem vorgegebenen Einnahmezeitintervall liegt und in dem vorgegebenen Einnahmezeitintervall eine der Arzneimittelportionen genommen worden ist, und wobei sich die zweite Zeitspanne maximal bis zum ersten dem zweiten Referenz-Einnahmezeitpunkt zeitlich nachfolgenden Referenz-Einnahmezeitpunkt erstreckt.
3. Anzeigeeinrichtung für einen Spender (Sp) für Arzneimittelportionen (Ta) nach Aspekt 2, dadurch gekennzeichnet, dass das zweite Visualisierungsmittel durch eine erste Entnahme einer der Arzneimittelportionen aus dem Spender in einem Einnahmezyklus gestartet wird.
4. Anzeigeeinrichtung für einen Spender (Sp) für Arzneimittelportionen (Ta) nach einem der Aspekte 2 - 4, dadurch gekennzeichnet, dass das erste Visualisierungsmittel durch Erreichen des ersten Referenz-Einnahmezeitpunktes gestartet wird, wenn bis zum ersten Referenz-Einnahmezeitpunkt keine der Arzneimittelportionen genommen worden ist.
5. Anzeigeeinrichtung für einen Spender (Sp) für Arzneimittelportionen (Ta) nach einem der Aspekte 2 - 5, dadurch gekennzeichnet, dass das erste Visualisierungsmittel in mindestens einem ersten Anzeigebereich und das zweite Visualisierungsmittel in mindestens einem zweiten Anzeigebereich der Anzeige (1) angeordnet sind.
6. Anzeigeeinrichtung für einen Spender (Sp) für Arzneimittelportionen (Ta) nach Aspekt 6, dadurch gekennzeichnet, dass der mindestens eine erste Anzeigebereich und der mindestens eine zweite Anzeigebereich zueinander benachbart sind.
7. Anzeigeeinrichtung für einen Spender (Sp) für Arzneimittelportionen (Ta) nach einem der vorstehenden Aspekte dadurch gekennzeichnet, dass das erste Visualisierungsmittel durch mindestens ein erstes Anzeigeelement (30, 40) gebildet ist.
8. Anzeigeeinrichtung für einen Spender (Sp) für Arzneimittelportionen (Ta) nach Aspekt 7, dadurch gekennzeichnet, dass mindestens zwei erste Anzeigeelemente (30, 40) vorgesehen sind und dass die ersten Anzeigeelemente mit fortschreitendem Zeitablauf sukzessive angeschaltet werden.
9. Anzeigevorrichtung für einen Spender (Sp) für Arzneimittelportionen (Ta) nach einem der vorstehenden Aspekte dadurch gekennzeichnet, dass das zweite Visualisierungsmittel durch mindestens ein zweites Anzeigeelement (20) gebildet ist.
10. Anzeigeeinrichtung für einen Spender (Sp) für Arzneimittelportionen (Ta) nach Aspekt 9, dadurch gekennzeichnet, dass mindestens zwei zweite Anzeigeelemente (20) vorgesehen sind und dass die zweiten Anzeigeelemente mit fortschreitendem Zeitablauf sukzessive angeschaltet werden.
11. Anzeigeeinrichtung für einen Spender (Sp) für Arzneimittelportionen (Ta) nach einem der Aspekte 7 - 10, dadurch gekennzeichnet, dass jedes des mindestens einen ersten Anzeigeelements (30, 40) und/oder jedes des mindestens einen zweiten Anzeigeelements (20) gleich großen Zeitabschnitten entspricht.
12. Anzeigeeinrichtung für einen Spender (Sp) für Arzneimittelportionen (Ta) nach Aspekt 11, dadurch gekennzeichnet, dass jedes des mindestens einen ersten Anzeigeelements (30, 40) und/oder jedes des mindestens einen zweiten Anzeigeelements (20) 1 Stunde entspricht.
13. Anzeigeeinrichtung für einen Spender (Sp) für Arzneimittelportionen (Ta) nach einem der Aspekte 7 - 12, dadurch gekennzeichnet, dass jedes des mindestens einen ersten Anzeigeelements (30, 40) ein Segment auf mindestens einem ersten Kreis bildet und/oder dass jedes des mindestens einen zweiten Anzeigeelements (20) ein Segment auf mindestens einem zweiten Kreis bildet.
14. Anzeigeeinrichtung für einen Spender (Sp) für Arzneimittelportionen (Ta) nach Aspekt 13, dadurch gekennzeichnet, dass sich der mindestens eine erste Kreis für das mindestens eine erste Anzeigeelement (30, 40) und der mindestens eine zweite Kreis für das mindestens eine zweite Anzeigeelement (20) zueinander konzentrisch sind.
15. Anzeigeeinrichtung für einen Spender (Sp) für Arzneimittelportionen (Ta) nach einem der vorstehenden Aspekte dadurch gekennzeichnet, dass das erste Visualisierungsmittel eine erste Zone und eine von der ersten Zone räumlich abgegrenzte zweite Zone umfasst, wobei die zweite Zone dazu dient, eine Überschreitung eines vorgegebenen dritten Zeitpunktes darzustellen.
16. Anzeigeeinrichtung für einen Spender (Sp) für Arzneimittelportionen (Ta) nach einem der vorstehenden Aspekte dadurch gekennzeichnet, dass zusätzlich eine Anzeige (50, 52) der Anzahl der sich in dem Spender befindenden Arzneimittelportionen umfasst ist.
17. Anzeigeeinrichtung für einen Spender (Sp) für Arzneimittelportionen (Ta) nach einem der vorstehenden Aspekte dadurch gekennzeichnet, dass zusätzlich eine Anzeige (50, 54) der Anzahl der in einem Einnahmezyklus bereits genommenen Arzneimittelportionen umfasst ist.
18. Anzeigeeinrichtung für einen Spender (Sp) für Arzneimittelportionen (Ta) nach einem der vorstehenden Aspekte , dadurch gekennzeichnet, dass zusätzlich eine Anzeige (60) für eine Einnahmeunterbrecnung umfasst ist.
19. Anzeigeeinrichtung für einen Spender (Sp) für Arzneimittelportionen (Ta) nach Aspekt 18, dadurch gekennzeichnet, dass die Anzeige (60) für die Einnahmeunterbrechung eine Darstellung (50) der Anzahl der Tage der Einnahmeunterbrechung einschließt.
20. Anzeigeeinrichtung für einen Spender (Sp) für Arzneimittelportionen (Ta) nach einem der Aspekte 18 und 19, dadurch gekennzeichnet, dass die ersten oder die zweiten Visualisierungsmittel während der Einnahmeunterbrechung die zwischen zwei Referenz-Einnahmezeitpunkten verstrichene Zeitspanne anzeigen.
21. Anzeigeeinrichtung für einen Spender (Sp) für Arzneimittelportionen (Ta) nach einem der vorstehenden Aspekte dadurch gekennzeichnet, dass die Anzeige (1) zusätzlich ein Anzeigeelement (82) für ein Warnsymbol wegen fehlender Kontrazeption umfasst.
22. Anzeigeeinrichtung für einen Spender (Sp) für Arzneimittelportionen (Ta) nach Aspekt 21, dadurch gekennzeichnet, dass das Warnsymbol auf der Anzeige (1) dargestellt wird, wenn folgende Bedingungen erfüllt sind:
   a) durch Versäumen einer Einnahme von mindestens einer Arzneimittelportion an mindestens einem Tag entsteht ein Fehleinnahme-Zeitraum, der dadurch definiert ist,
      i) dass sich der Fehleinnahme-Zeitraum an einen ersten Einnahme-Zeitraum von mindestens 7 Tagen mit ununterbrochener Einnahme von Arzneimittelportionen anschließt,
      ii) dass sich an den Fehleinnahme-Zeitraum wiederum ein zweiter Einnahme-Zeitraum von mindestens 7 Tagen mit geforderter ununterbrochener Einnahme von Arzneimittelportionen anschließt,
      iii) wobei an mindestens einem Tag in dem Fehleinnahme-Zeitraum keine Arzneimittelportion genommen wird,
      iv) dass innerhalb des Fehleinnahme-Zeitraumes kein Zeitraum mit mindestens 7-tägiger ununterbrochener Einnahme eingeschlossen ist,
      v) das an dem ersten und letzten Tag des Fehleinnahme-Zeitraumeskeine Arzneimittelportion genommen wird und
      vi) dass der Fehleinnahme-Zeitraum länger als 7 Tage andauert;
   b) der erste Tag, an dem das Warnsymbol erscheint, ist der 8. Tag des Fehleinnahme-Zeitraumes;
   c) der letzte Tag, an dem das Warnsymbol erscheint, ist der 7. Tag des sich an den Fehleinnahme-Zeitraum anschließenden zweiten Einnahme-Zeitraumes mit ununterbrochener Einnahme von Arzneimittelportionen.
23. Anzeigeeinrichtung für einen Spender (Sp) für Arzneimittelportionen (Ta) nach einem der vorstehenden Aspekte dadurch gekennzeichnet, dass die Anzeigeeinrichtung eine digitale Anzeigeeinrichtung ist.
24. Anzeigeeinrichtung für einen Spender (Sp) für Arzneimittelportionen (Ta) nach einem der vorstehenden Aspekte , dadurch gekennzeichnet, dass die Anzeigeeinrichtung eine LCD-Anzeigeeinrichtung ist.
25. Verwendung der Anzeigeeinrichtung nach einem der Aspekte 1 - 24 zur Kontrolle der Einnahme von Arzneimittelportionen (Ta) aus einem Spender (Sp).
26. Verwendung nach Aspekt 25, dadurch gekennzeichnet, dass die Arzneimittelportionen (Ta) Hormonpräparate-Portionen sind.
27. Verwendung nach einem der Aspekte 25 und 26, dadurch gekennzeichnet, dass die Arzneimittelportionen (Ta) Kontrazeptiva-Portionen sind.
28. Verwendung nach Aspekt 27, dadurch gekennzeichnet, dass die Kontrazeptiva-Portionen in einer Einnahmephase genommen werden und dass sich an die Einnahmephase eine Phase einer Einnahmeunterbrechung anschließt.
29. Verwendung nach Aspekt 28, dadurch gekennzeichnet, dass die Einnahmephase einen ersten Einnahme-Zeitraum, der nicht durch die Phase der Einnahmeunterbrechung unterbrochen werden darf, und einen zweiten Einnahme-Zeitraum, der durch die Phase der Einnahmeunterbrechung unterbrochen werden darf, umfasst.

## Patentansprüche

1. Anzeigeeinrichtung für einen Spender (Sp) für Arzneimittelportionen (Ta), wobei die Arzneimittelportionen zu regelmäßig wiederkehrenden Referenz-Einnahmezeitpunkten einzunehmen sind und wobei die Anzeigeeinrichtung eine Anzeige (1) sowie eine elektronische Ansteuerung für die Anzeige umfasst, **dadurch gekennzeichnet, dass** die Anzeige ein erstes Visualisierungsmittel zur Darstellung einer ersten Zeitspanne zwischen einem ersten Referenz-Einnahmezeitpunkt und dem aktuellen Zeitpunkt umfasst und sich der erste Referenz-Einnahmezeitpunkt dadurch auszeichnet, dass bis zu dem ersten Referenz-Einnahmezeitpunkt keine Arzneimittelportion genommen worden ist.

2. Anzeigeeinrichtung für einen Spender (Sp) für Arzneimittelportionen (Ta) nach Anspruch 1, **dadurch gekennzeichnet, dass** die Anzeige (1) zusätzlich ein zweites Visualisierungsmittel zur Darstellung einer zweiten Zeitspanne zwischen einem zweiten Referenz-Einnahmezeitpunkt und dem aktuellen Zeitpunkt umfasst, wobei sich der zweite Referenz-Einnahmezeitpunkt dadurch auszeichnet, dass er in einem vorgegebenen Einnahmezeitintervall liegt und in dem vorgegebenen Einnahmezeitintervall eine der Arzneimittelportionen genommen worden ist, und wobei sich die zweite Zeitspanne maximal bis zum ersten dem zweiten Referenz-Einnahmezeitpunkt zeitlich nachfolgenden Referenz-Einnahmezeitpunkt erstreckt, und dass das zweite Visualisierungsmittel durch eine erste Entnahme einer der Arzneimittelportionen aus dem Spender in einem Einnahmezyklus gestartet wird.

3. Anzeigeeinrichtung für einen Spender (Sp) für Arzneimittelportionen (Ta) nach Anspruch 2, **dadurch gekennzeichnet, dass** das erste Visualisierungsmittel durch Erreichen des ersten Referenz-Einnahmezeitpunktes gestartet wird, wenn bis zum ersten Referenz-Einnahmezeitpunkt keine der Arzneimittelportionen genommen worden ist.

4. Anzeigeeinrichtung für einen Spender (Sp) für Arzneimittelportionen (Ta) nach einem der Ansprüche 2 oder 3, **dadurch gekennzeichnet, dass** das erste Visualisierungsmittel in mindestens einem ersten Anzeigebereich und das zweite Visualisierungsmittel in mindestens einem zweiten Anzeigebereich der Anzeige (1) angeordnet sind.

5. Anzeigeeinrichtung für einen Spender (Sp) für Arzneimittelportionen (Ta) nach einem der vorstehenden Ansprüche, **dadurch gekennzeichnet, dass** das erste Visualisierungsmittel durch mindestens zwei erste Anzeigeelemente (30, 40) gebildet wird und dass die ersten Anzeigeelemente mit fortschreitendem Zeitablauf sukzessive angeschaltet werden.

6. Anzeigevorrichtung für einen Spender (Sp) für Arzneimittelportionen (Ta) nach einem der vorstehenden Ansprüche, **dadurch gekennzeichnet, dass** das zweite Visualisierungsmittel durch mindestens zwei zweite Anzeigeelemente (20) gebildet wird und dass die zweiten Anzeigeelemente mit fortschreitendem Zeitablauf sukzessive angeschaltet werden.

7. Anzeigeeinrichtung für einen Spender (Sp) für Arzneimittelportionen (Ta) nach einem der Ansprüche 5 oder 6, **dadurch gekennzeichnet, dass** jedes des mindestens einen ersten Anzeigeelements (30, 40) und/oder jedes des mindestens einen zweiten Anzeigeelements (20) gleich großen Zeitabschnitten entspricht.

8. Anzeigeeinrichtung für einen Spender (Sp) für Arzneimittelportionen (Ta) nach einem der vorstehenden Ansprüche, **dadurch gekennzeichnet, dass** das erste Visualisierungsmittel eine erste Zone und eine von der ersten Zone räumlich abgegrenzte zweite Zone umfasst, wobei die zweite Zone dazu dient, eine Überschreitung eines vorgegebenen dritten Zeitpunktes darzustellen.

9. Anzeigeeinrichtung für einen Spender (Sp) für Arzneimittelportionen (Ta) nach einem der vorstehenden Ansprüche, **dadurch gekennzeichnet, dass** zusätzlich eine Anzeige (50, 52) der Anzahl der sich in dem Spender befindenden Arzneimittelportionen umfasst ist.

10. Anzeigeeinrichtung für einen Spender (Sp) für Arzneimittelportionen (Ta) nach einem der vorstehenden Ansprüche, **dadurch gekennzeichnet, dass** zusätzlich eine Anzeige (50, 54) der Anzahl der in einem Einnahmezyklus bereits genommenen Arzneimittelportionen umfasst ist.

11. Anzeigeeinrichtung für einen Spender (Sp) für Arzneimittelportionen (Ta) nach Anspruch 10, **dadurch gekennzeichnet, dass** zusätzlich eine Anzeige (60) für eine Einnahmeunterbrechung umfasst ist, und dass die Anzeige (60) für die Einnahmeunterbrechung eine Darstellung (50) der Anzahl der Tage der Einnahmeunterbrechung einschließt.

12. Anzeigeeinrichtung für einen Spender (Sp) für Arzneimittelportionen (Ta) nach Anspruch 11, **dadurch gekennzeichnet, dass** die ersten oder die zweiten Visualisierungsmittel während der Einnahmeunterbrechung die zwischen zwei Referenz-Einnahmezeitpunkten verstrichene Zeitspanne anzeigen.

13. Anzeigeeinrichtung für einen Spender (Sp) für Arzneimittelportionen (Ta) nach einem der vorstehenden Ansprüche, **dadurch gekennzeichnet, dass** die Anzeigeeinrichtung eine digitale Anzeigeeinrichtung ist.

14. Anzeigeeinrichtung für einen Spender (Sp) für Arzneimittelportionen (Ta) nach einem der vorstehenden Ansprüche, **dadurch gekennzeichnet, dass** die Anzeigeeinrichtung eine LCD-Anzeigeeinrichtung ist.

15. Verwendung der Anzeigeeinrichtung nach einem der Ansprüche 1 - 14 zur Kontrolle der Einnahme von Arzneimittelportionen (Ta) aus einem Spender (Sp).
